(19) Europäisches Patentamt
European Patent Office
Office européen des brevets

(11) EP 3 177 735 B1

(12) FASCICULE DE BREVET EUROPEEN

(45) Date de publication et mention
de la délivrance du brevet:
**25.09.2019 Bulletin 2019/39**

(21) Numéro de dépôt: **15759894.7**

(22) Date de dépôt: **06.08.2015**

(51) Int Cl.:
***G01N 33/50*** (2006.01)   ***C12Q 1/6883*** (2018.01)

(86) Numéro de dépôt international:
**PCT/FR2015/052171**

(87) Numéro de publication internationale:
**WO 2016/020625 (11.02.2016 Gazette 2016/06)**

(54) **MICROARNS CARACTÉRISANT LA ROSACÉE DE TYPE II ET LEURS UTILISATIONS**

MIKRORNAS ZUR CHARAKTERISIERUNG VON ROSACEA TYPE II UND VERWENDUNGEN DAVON

MICRORNAS CHARACTERISING ROSACEA TYPE II AND THE USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.08.2014 FR 1457668**

(43) Date de publication de la demande:
**14.06.2017 Bulletin 2017/24**

(73) Titulaire: **Galderma Research & Development 06410 Biot (FR)**

(72) Inventeurs:
• **MOUNIER, Carine**
  **F-06560 Valbonne (FR)**
• **DERET, Sophie**
  **F-06250 Mougins (FR)**

(74) Mandataire: **Nederlandsch Octrooibureau P.O. Box 29720 2502 LS The Hague (NL)**

(56) Documents cités:
**WO-A2-2005/118806   WO-A2-2011/053257**

• **Genechip: "Data Sheet GeneChip TM miRNA 3.0 Array", , 29 mars 2012 (2012-03-29), XP055222758, Extrait de l'Internet: URL:http://www.carrerasresearch.org/genech ip-mirna-3-0-array_38713.pdf [extrait le 2015-10-21]**

**Description**

La présente invention relève du domaine de la médecine, et plus particulièrement du diagnostic et du traitement de la rosacée.

**ART ANTERIEUR**

Le nom de rosacée fait référence à la couleur caractéristique que prend le visage du patient. Bien qu'elle n'ait pas de conséquences majeures sur la santé de l'individu, la rosacée peut avoir des conséquences psycho-affectives importantes.

La rosacée débute rarement avant l'âge de trente ans, avec une fréquence qui augmente progressivement jusqu'à un pic autour de 40/50 ans. Une étude a mis en évidence une fréquence plus importante du sous-type I (environ 45% des patients atteints de rosacée) par rapport au sous-type II (environ 25%). Par ailleurs, la rosacée de type I toucherait majoritairement les femmes (environ 2 tiers des patients) tandis que pour les formes de rosacée de type II il n'a pas été montré de différences de distribution selon le genre.

La rosacée est associée à tort à la consommation excessive d'alcool ou à l'acné. Malgré sa fréquence, ses causes en restent encore mal déterminées. L'alimentation et les facteurs climatiques jouent un rôle dans la maladie et ont un impact sur ses symptômes. Bien que la rosacée soit connue de longue date et touche de nombreux patients, ses causes ne sont pas élucidées à ce jour. Un caractère de prédisposition génétique est suspecté: 40% des individus touchés par une forme de rosacée possède ont un membre de leur famille également atteint.

De nombreux facteurs sont considérés comme favorisant/déclenchant l'apparition de la rosacée tels que : un stress émotionnel, les boissons chaudes, l'alcool, la nourriture épicée, l'exercice physique, les températures extrêmes et les changements brutaux de température, les bains/douches chaud(e)s, etc.

Tous ces facteurs vont avoir tendance à favoriser les bouffés vasomotrices, favorisant le déclenchement de la pathologie.

La rosacée est caractérisée par :

- Des problèmes vasculaires et vasomoteurs avec notamment une vasodilatation des vaisseaux favorisant l'apparition de télangiectasies.
- Une inflammation qui se traduit par une réaction immunitaire avec le recrutement des cellules de l'immunité innée (macrophages, neutrophiles, cellules dendritiques) et de l'immunité acquise (lymphocytes Th1, lymphocytes B sécréteurs d'anticorps, cellules T cytotoxiques). Au niveau moléculaire, Cette inflammation est exacerbée dans le sous-type II.Un relâchement du tissu conjonctif lâche de la peau (élastose actinique), retrouvée également dans les phénomènes de photovieillissement et qui pourrait également être due à l'âge des patients.

La rosacée Erythémato-télangiectasique (ETR) ou rosacée de type I (RI) est la forme la plus fréquente caractérisée par une rougeur persistante, ou érythème. Elle se concentre généralement au centre du visage en épargnant le pourtour des yeux et celui de la bouche mais en incluant les joues, le nez, le milieu du front et le menton. Dans cette forme, la rougeur est accompagnée d'une sensibilité exacerbée de la peau qui rend difficile l'application de cosmétiques. La coloration de la peau peut être associée au développement de petits vaisseaux très fins, très rouges et parfois même violacés visibles sous la surface de la peau : les télangiectasies. Cette forme s'accompagne également souvent de manifestations type de bouffées de chaleur.

Dans la Rosacée papulopustuleuse (PPR) ou rosacée de type II (RII), des papules et/ou pustules, ressemblant à des lésions d'acné, peuvent apparaître sur l'érythème, d'où le nom de rosacée papulopustuleuse. Les *papules* sont des élévations de la peau, rouges, fermes et parfois douloureuses qui mesurent de un à quatre millimètres, et qui sont entourées d'une auréole inflammatoire. Elles expriment parfois l'invasion de la glande sébacée par un parasite appelé *Demodex folliculorum,* présent habituellement dans le follicule. Les *pustules* ont une taille souvent inférieure à celle des papules et peuvent se développer en dehors de tout contexte infectieux.

Ainsi, il reste difficile de distinguer un acné de type acne vulgaris, d'une rosacée, en particulier d'une rosacée de type II.

**RESUME DE L'INVENTION**

La présente invention est décrite dans les revendications ci-jointes. Elle présente invention fournit une méthode pour diagnostiquer une rosacée de type II, chez un sujet, comprenant la détermination dans un échantillon de derme et/ou épiderme dudit sujet de l'expression d'au moins la combinaison des sept microARNs suivants : hsa-miR-3201, hsa-miR-4423-3p, hsa-miR-3128, hsa-miR-3163, hsa-miR-606, hsa-miR-4776-5p, et hsa-miR-635.

Ces microARNs sont donc utiles comme biomarqueurs de la rosacée. Plus précisément, il s'agit de biomarqueurs spécifiques de la rosacée de type II, dont l'expression n'est pas altérée ni chez des sujets normaux, ni chez des sujets atteints de rosacée de type I. De même, l'expression de ces microARNs n'est pas non plus altérée chez les patients souffrant d'acné, bien que ces deux pathologies soient difficiles à distinguer.

Il est également décrit un kit comprenant des moyens de détection spécifiques de ces sept microARNs suivants, et son utilisation pour le diagnostic de la rosacée, pour le criblage de molécules susceptibles d'être utilisées pour le traitement de la rosacée, pour distinguer la rosacée de type II de la rosacée de type I, ou pour déterminer l'efficacité d'un traitement de la rosacée, de préférence la rosacée de type II.

## DESCRIPTION DE L'INVENTION

*Définitions*

*MicroARNs*

Les microARNs sont de petits ARNs non codants de 18 à 25 nucléotides, exprimés chez la plupart des organismes eucaryotes, et qui jouent un rôle important dans la régulation de l'expression des gènes. Ce sont de puissants répresseurs post-transcriptionnels : en se fixant par complémentarité à des séquences nucléotidiques spécifiques présentes sur des ARNm, ils vont ainsi empêcher la traduction en protéines de ces transcrits dits « cibles ». Un microARN possède plusieurs ARNm cibles et réciproquement, un ARNm est la cible de plusieurs microARNs.

Tous les microARNs connus sont référencés dans la base miRBase (miRBase, http://www.mirbase.org). Des microARNs homologues peuvent être retrouvés dans plusieurs organismes ; un système d'annotation a donc été mis en place pour leur attribuer un identifiant unique. Les microARNs sont identifiés par un numéro précédé de l'abréviation « miR » ou « mir », qui permet une distinction entre le microARN mature (miR) et la structure tige-boucle du précurseur de microARN (mir). Un préfixe est utilisé pour distinguer les espèces comme par exemple hsa-miR-101 et mmu-miR-101 pour faire une différence entre le microARN chez l'Homme (hsa : *Homo sapiens*) et chez la Souris (mmu : *Mus musculus*). Dans certains cas, un même précurseur tige-boucle peut donner lieu à la synthèse de deux microARNs différents. Le système d'annotation permet la distinction de ces deux microARNs : le microARN du côté 5' de la boucle est noté 5p et le microARN du côté 3' est noté 3p. Cette annotation 3p et 5p est utilisée jusqu'à ce que l'abondance de l'une des deux formes soit déterminée. La forme majoritaire « miR-xxx » se distingue alors de la forme minoritaire « miR-xxx* ».

Les microARNs sont impliqués dans une large gamme de processus biologiques clés tels que le contrôle du cycle cellulaire et l'apoptose. Ils régulent également plusieurs processus physiologiques et de développement comme la différenciation des cellules souches, l'hématopoïèse, l'hypoxie, le développement musculaire, la neurogenèse, la sécrétion d'insuline, le métabolisme du cholestérol, le vieillissement, les réponses immunitaires et inflammatoires. En outre, des schémas distincts d'expression temporelle au cours de l'embryogenèse ainsi que des profils d'expression spécifiques par tissu suggèrent que les microARNs jouent un rôle essentiel dans la différenciation et le maintien de l'identité des tissus. Ces processus biologiques sont souvent déréglés chez des individus malades. De nombreuses études ont mis en évidence l'implication des microARNs dans certaines pathologies, dont différents cancers, des maladies cardiaques et des maladies neurologiques. Plus récemment, le rôle des microARNs a également été démontré dans certaines maladies dermatologiques telles que le psoriasis, le vitiligo ou encore certains cancers de la peau.

*Sujets*

Les sujets sont des patients humains, homme ou femme, de tout âge, de préférence de plus de 18 ans.

*Echantillons biologiques*

Les échantillons testés sont typiquement des échantillons de derme, d'épiderme ou de derme et d'épiderme. Dans certains modes de réalisation, il s'agit d'une biopsie de peau, de préférence prélevées au niveau des zones de la peau atteintes. Il peut aussi s'agir de tout échantillon de liquide biologique, comme du sang, de la salive, de l'urine.

*Contrôles*

Les échantillons contrôles, ou des niveaux d'expression contrôles, sont des échantillons, ou des niveaux d'expression, issus de sujets sains ou de sujets atteints d'une autre pathologie, éventuellement une autre pathologie cutanée.

*Applications diagnostiques*

La présente invention est relative à l'identification de microARNs et/ou de précurseurs de ceux-ci qui sont différentiellement exprimés chez les sujets souffrant de rosacée par rapport à des sujets sains ou par rapport à des sujets atteints d'une autre pathologie cutanée, comme l'acné. En particulier, elle est relative à l'identification de microARNs et/ou de précurseurs de ceux-ci qui sont différentiellement exprimés chez les sujets souffrant de la rosacée de type II par rapport à des sujets sains et/ou des sujets souffrant de la rosacée de type I.

Sur la base des microARNs identifiés, il est possible de :

- utiliser ces microARNs comme biomarqueurs de la rosacée ou de sous-type de la rosacée, en particulier le sous-type II ;
- utiliser d'un ou plusieurs des microARNs identifiés pour le diagnostic, la détection, la détermination du sous-type, le suivie et le pronostic de la rosacée ;
- réaliser une méthode de diagnostic, de détection, de détermination du sous-type, de suivi et de pronostic de la rosacée, la méthode comprenant au moins une étape de détermination du niveau d'expression d'un ou plusieurs des microARNs identifiés dans un échantillon d'un patient.
- Préparer un kit de diagnostic, de détection, de détermination du sous-type, de suivi et de pronostic de la rosacée, le kit comprenant des moyens de détection du niveau d'expression d'un ou plusieurs des microARNs identifiés. De préférence, les moyens de détection sont des acides nucléiques ou des peptides présentant une capacité à se lier à un ou plusieurs des microARNs identifiés, de préférence des oligonucléotides ou sondes spécifiques d'un ou plusieurs des microARNs identifiés
- Réaliser une méthode de suivi de l'efficacité thérapeutique d'un traitement dans laquelle une étape de détermination du niveau d'expression d'un ou plusieurs des microARNs identifiés dans un échantillon d'un patient est réalisée avant, pendant et/ou après traitement, et les niveaux d'expression sont comparés.
- utiliser ces microARNs pour effectuer des analyses de populations ou des clusterings, par exemple pour différentier les sujets souffrant de la rosacée de type I et ceux de la rosacée de sous-type II, les sujets souffrant de la rosacée et les sujets sains, ou encore les sujets souffrant de la rosacée de type II et les sujets sains.
- utiliser ces microARNs pour effectuer des analyses de populations ou des clusterings, par exemple pour différentier les sujets souffrant de la rosacée et les sujets souffrant d'acné.

Une fois le diagnostic posé, un traitement de la rosacée peut être envisagé. Lorsqu'une rosacée de type II a été diagnostiquée, un traitement avec par exemple de la doxycycline (par voie orale), ou avec du métronidazole ou de l'ivermectine topique, peut être prescrit.

Un objet de l'invention concerne donc une méthode pour diagnostiquer une rosacée de type II chez un sujet, comprenant la détermination dans un échantillon de derme et/ou épiderme dudit sujet de l'expression d'au moins la combinaison des sept microARNs suivants : hsa-miR-3201, hsa-miR-4423-3p, hsa-miR-3128, hsa-miR-3163, hsa-miR-606, hsa-miR-4776-5p, et hsa-miR-635. On considère que le patient souffre de rosacée si l'expression de ces microARNs est supérieure à celle des sujets contrôles.

De manière avantageuse, la méthode peut comprendre la détermination supplémentaire de l'expression d'un ou plusieurs microARNs choisis parmi

| | |
|---|---|
| hsa-miR-133a | hsa-miR-3201 |
| hsa-miR-133b | hsa-miR-4423-3p |
| hsa-miR-1 | hsa-miR-3128 |
| hsa-miR-299-5p | hsa-miR-155 |
| hsa-miR-486-3p | hsa-miR-3163 |
| hsa-miR-381 | hsa-miR-146b-5p |
| hsa-miR-4324 | hsa-miR-606 |
| hsa-miR-154 | hsa-miR-150 |
| hsa-miR-1247 | hsa-miR-4776-5p |
| hsa-miR-1287 | hsa-miR-3124-5p |
| hsa-miR-376c | hsa-miR-4741 |
| hsa-miR-195* | hsa-miR-635 |
| hsa-miR-4269 | hsa-miR-4668-5p |
| hsa-miR-1296 | hsa-miR-4763-3p |
| hsa-miR-34c-3p | hsa-miR-4417 |

(suite)

| | |
|---|---|
| hsa-miR-204 | hsa-miR-1911* |
| hsa-miR-504 | hsa-miR-4734 |
| hsa-miR-30c-1* | hsa-miR-4530 |
| hsa-miR-615-3p | hsa-miR-4260 |
| hsa-miR-505 | hsa-miR-3185 |
| hsa-miR-508-5p | hsa-miR-4707-5p |
| hsa-miR-409-5p | hsa-miR-4745-5p |
| hsa-miR-433 | hsa-miR-4659a-3p |
| hsa-miR-375 | hsa-miR-1469 |
| hsa-miR-935 | hsa-miR-4695-5p |
| hsa-miR-128 | hsa-miR-4674 |
| hsa-miR-331-5p | hsa-miR-4687-3p |
| hsa-miR-584 | hsa-miR-4739 |
| hsa-miR-30a* | hsa-miR-1915 |
| hsa-miR-370 | hsa-miR-216b |
| hsa-miR-148a* | hsa-miR-548a-3p |
| hsa-miR-489 | |
| hsa-miR-30e* | |
| hsa-miR-337-5p | |
| hsa-miR-1181 | |
| hsa-miR-1237 | |
| hsa-miR-485-5p | |
| hsa-miR-23c | |
| hsa-miR-148b | |
| hsa-miR-378g | |
| hsa-miR-1238 | |
| hsa-miR-181a-2* | |
| hsa-miR-187 | |
| hsa-miR-378d | |
| hsa-miR-181d | |
| hsa-miR-98 | |
| hsa-miR-4685-3p | |
| hsa-miR-23b* | |

Un niveau d'expression altéré d'au moins un de ces microARNs par rapport au niveau d'expression chez des sujets contrôles renforce le diagnostic.

Selon un aspect particulier, l'expression d'un ou plusieurs microARNs est en outre déterminée, lesdits ARNs étant choisis dans un des groupes consistant en

1) hsa-miR-133a, hsa-miR-206, hsa-miR-133b, hsa-miR-1, hsa-miR-299-5p, hsa-miR-486-3p, hsa-miR-381, hsa-miR-4324, hsa-miR-154, hsa-miR-1247, hsa-miR-1287, hsa-miR-376c, hsa-miR-195*, hsa-miR-411, hsa-miR-4269, hsa-miR-328, hsa-miR-1296, hsa-miR-34c-3p, hsa-miR-204, hsa-miR-504, hsa-miR-30c-1*, hsa-miR-615-3p, hsa-miR-654-3p, hsa-miR-505, hsa-miR-508-5p, hsa-miR-409-5p, hsa-miR-338-5p, hsa-miR-433, hsa-miR-375, hsa-miR-935, hsa-let-7d*, hsa-miR-99a*, hsa-miR-128, hsa-miR-1290, hsa-miR-331-5p, hsa-miR-4730, hsa-miR-29c*, hsa-miR-455-5p, hsa-miR-377*, hsa-miR-378e, hsa-miR-584, hsa-miR-96, hsa-miR-30a*, hsa-miR-143*, hsa-miR-370, hsa-miR-148a*, hsa-miR-489, hsa-miR-506, hsa-let-7e*, hsa-miR-30e*, hsa-miR-4787-3p, hsa-let-7b*, hsa-miR-181c*, hsa-miR-513a-5p, hsa-miR-92a-1*, hsa-miR-337-5p, hsa-miR-1181, hsa-miR-1237, hsa-miR-3620, hsa-miR-485-5p, hsa-miR-23c,, , hsa-miR-18a*, hsa-miR-10a, hsa-miR-139-3p, hsa-miR-493, hsa-miR-148b, hsa-miR-550a, hsa-miR-3147, hsa-miR-378g, hsa-miR-675*, hsa-miR-1238, hsa-miR-542-5p, hsa-miR-30c-2*, hsa-miR-125a-3p, hsa-miR-181a-2*, hsa-miR-1292, hsa-miR-187, hsa-miR-378*, hsa-miR-493*, hsa-miR-495, hsa-miR-557, hsa-miR-3909, hsa-miR-378d, hsa-miR-491-5p, hsa-miR-181d, hsa-miR-671-3p, hsa-miR-513c, hsa-miR-487b, hsa-miR-378b, hsa-miR-885-5p, hsa-miR-98, hsa-miR-29b-2*, hsa-miR-4685-3p, hsa-miR-3605-3p, hsa-miR-24-1*, hsa-miR-4649-3p, hsa-miR-3180-5p, hsa-miR-149, hsa-miR-23b*, hsa-miR-4758-5p, hsa-miR-4665-5p, hsa-miR-149*, hsa-miR-548ac, hsa-miR-4507, hsa-miR-548a-3p, hsa-miR-4289, hsa-miR-4727-3p, hsa-miR-4468, hsa-miR-4463, hsa-miR-2861, hsa-miR-4773, hsa-miR-1825, hsa-miR-4651, hsa-miR-216b, hsa-miR-4689, hsa-miR-3152-3p, hsa-miR-4270, hsa-miR-1915, hsa-miR-4739, hsa-miR-4772-5p, hsa-miR-4687-3p, hsa-miR-4674, hsa-miR-4695-5p, hsa-miR-4439, hsa-miR-129-3p, hsa-miR-1469, hsa-miR-4659a-3p, hsa-miR-4799-3p, hsa-miR-4657, hsa-miR-4655-5p, hsa-miR-4745-5p, hsa-miR-4662b, hsa-miR-4707-5p, hsa-miR-3185, hsa-miR-4260, hsa-miR-4530, hsa-miR-4734, hsa-miR-1911*, hsa-miR-4417, hsa-miR-4763-3p, hsa-miR-4668-5p, hsa-miR-1281, hsa-miR-635, hsa-miR-4741, hsa-miR-3124-5p, hsa-miR-146b-3p, hsa-miR-4529-3p, hsa-miR-4776-5p, hsa-miR-150, hsa-miR-3927, hsa-miR-606, hsa-miR-146b-5p, hsa-miR-3163, hsa-miR-155, hsa-miR-371b-5p, hsa-miR-3128, hsa-miR-4423-3p, hsa-miR-335, hsa-miR-3201, hsa-miR-184

2) hsa-miR-206, hsa-miR-133b, hsa-miR-133a, hsa-miR-1, hsa-miR-486-3p, hsa-miR-299-5p, hsa-miR-1247, hsa-miR-381, hsa-miR-154, hsa-miR-433, hsa-miR-1244, hsa-miR-4485, hsa-miR-378e, hsa-miR-505, hsa-miR-376c, hsa-miR-1296, hsa-miR-29b-1*, hsa-miR-409-5p, hsa-miR-29c*, hsa-miR-34c-3p, hsa-miR-4646-5p, hsa-miR-99a*, hsa-miR-378g, hsa-miR-504, hsa-miR-584, hsa-miR-128, hsa-miR-30c-1*, hsa-miR-665, hsa-miR-4269, hsa-miR-493*, hsa-miR-328, hsa-miR-550a, hsa-miR-375, hsa-miR-493, hsa-miR-378d, hsa-miR-148a*, hsa-miR-615-3p, hsa-miR-431*, hsa-miR-378i, hsa-miR-422a, hsa-miR-331-5p, hsa-miR-378*, hsa-miR-148b, hsa-miR-601, hsa-miR-4288, hsa-miR-596, hsa-miR-10a, hsa-miR-200c*, hsa-miR-378b, hsa-miR-378f, hsa-miR-4257, hsa-miR-370, hsa-miR-1237, hsa-miR-3942-3p, hsa-miR-1269b, hsa-miR-4741, hsa-miR-4763-3p, hsa-miR-4704-5p, hsa-miR-1911*, hsa-miR-3621, hsa-miR-3612, hsa-miR-4436b-5p, hsa-miR-4734, hsa-miR-3185, hsa-miR-3910, hsa-miR-4727-3p, hsa-miR-155, hsa-miR-155*, hsa-miR-4529-3p, hsa-miR-3927, hsa-miR-3201, hsa-miR-4423-3p, hsa-miR-124, et hsa-miR-184;

3) hsa-miR-211, hsa-miR-29b, hsa-miR-4324, hsa-miR-143*, hsa-miR-1287, hsa-miR-4708-5p, hsa-miR-195*, hsa-miR-508-5p, hsa-miR-204, hsa-miR-96, hsa-let-7b*, hsa-miR-935, hsa-miR-675*, hsa-miR-149, hsa-miR-30a*, hsa-miR-1181, hsa-miR-506, hsa-miR-23c, hsa-miR-127-5p, hsa-miR-491-5p, hsa-miR-4776-5p, hsa-miR-601, hsa-miR-4530, hsa-miR-4773, hsa-miR-4717-3p, hsa-miR-4657, hsa-miR-4289, hsa-miR-4417, hsa-miR-4445*, hsa-miR-150, hsa-miR-146b-5p, hsa-miR-3175, hsa-miR-4646-5p, hsa-miR-3163, hsa-miR-4423-3p, hsa-miR-3128, hsa-miR-1244, hsa-miR-21*, hsa-miR-3201, hsa-miR-335

4) hsa-miR-223, hsa-miR-142-3p, hsa-miR-146b, hsa-miR-21, hsa-miR-155, hsa-miR-30b, hsa-miR-221, hsa-miR-141, hsa-miR-339-3p, hsa-miR-100, hsa-miR-331, hsa-miR-598, hsa-miR-24, hsa-miR-23a, hsa-miR-99a, hsa-miR-152, hsa-miR-423-5p, hsa-miR-34c, hsa-miR-27a, hsa-miR-30c, hsa-miR-199a, hsa-miR-125a-5p, mmu-miR-491, hsa-miR-489, hsa-miR-337-5p, hsa-miR-149, hsa-miR-127, hsa-miR-375, hsa-miR-101, hsa-miR-296, mmu-miR-379, hsa-miR-574-3p, hsa-miR-487b, mmu-miR-499, hsa-miR-95, hsa-miR-885-5p, hsa-miR-486-3p, hsa-miR-1, hsa-miR-133b, hsa-miR-133a.

Selon un autre aspect particulier, l'expression d'un ou plusieurs microARNs est en outre déterminée, lesdits ARNs étant choisis dans un des groupes consistant en

a) hsa-miR-133a, hsa-miR-133b, hsa-miR-1, hsa-miR-299-5p, hsa-miR-486-3p, hsa-miR-381, hsa-miR-4324, hsa-miR-154, hsa-miR-1247, hsa-miR-1287, hsa-miR-376c, hsa-miR-195*, hsa-miR-4269, hsa-miR-1296, hsa-miR-34c-3p, hsa-miR-204, hsa-miR-504, hsa-miR-30c-1*, hsa-miR-615-3p, hsa-miR-505, hsa-miR-508-5p, hsa-miR-409-5p, hsa-miR-433, hsa-miR-375, hsa-miR-935, hsa-miR-128, hsa-miR-331-5p, hsa-miR-584, hsa-miR-30a*, hsa-miR-370, hsa-miR-148a*, hsa-miR-489, hsa-miR-30e*, hsa-miR-337-5p, hsa-miR-1181, hsa-miR-1237, hsa-miR-485-5p, hsa-miR-23c, hsa-miR-148b, hsa-miR-378g, hsa-miR-1238, hsa-miR-181a-2*, hsa-miR-187, hsa-miR-378d, hsa-miR-181d, hsa-miR-98, hsa-miR-4685-3p, hsa-miR-23b*, hsa-miR-3201, hsa-miR-4423-3p, hsa-miR-3128, hsa-miR-155, hsa-miR-3163, hsa-miR-146b-5p, hsa-miR-606, hsa-miR-150, hsa-miR-4776-5p, hsa-miR-3124-5p, hsa-miR-4741, hsa-miR-635, hsa-miR-4668-5p, hsa-miR-4763-3p, hsa-miR-4417, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

b) hsa-miR-223, hsa-miR-142-3p, hsa-miR-146b, hsa-miR-21, hsa-miR-331, hsa-miR-598, hsa-miR-24, hsa-miR-23a, hsa-miR-99a, hsa-miR-152, hsa-miR-423-5p, hsa-miR-34c, hsa-miR-27a, hsa-miR-30c, hsa-miR-125a-5p, mmu-miR-491, hsa-miR-127, hsa-miR-375, hsa-miR-101, hsa-miR-296, mmu-miR-379, hsa-miR-574-3p, hsa-miR-487b, mmu-miR-499, hsa-miR-95, hsa-miR-885-5p, hsa-miR-486-3p, hsa-miR-1, hsa-miR-133b, hsa-miR-133a

c) hsa-miR-133a, hsa-miR-133b, hsa-miR-1, hsa-miR-299-5p, hsa-miR-486-3p, hsa-miR-381, hsa-miR-4324, hsa-miR-154, hsa-miR-1247, hsa-miR-885-5p, hsa-miR-1287, hsa-miR-95, hsa-miR-376c, hsa-miR-195*, hsa-miR-4269, mmu-miR-499, hsa-miR-1296, hsa-miR-34c-3p, hsa-miR-204, hsa-miR-504, hsa-miR-30c-1*, hsa-miR-487b, hsa-miR-615-3p, hsa-miR-505, hsa-miR-508-5p, hsa-miR-409-5p, hsa-miR-433, hsa-miR-375, hsa-miR-935, hsa-miR-128, hsa-miR-331-5p, hsa-miR-584, hsa-miR-30a*, hsa-miR-370, hsa-miR-148a*, hsa-miR-489, hsa-miR-30e*, hsa-miR-337-5p, hsa-miR-1181, hsa-miR-1237, hsa-miR-485-5p, hsa-miR-23c, hsa-miR-491-5p, hsa-miR-148b, hsa-miR-378g, hsa-miR-125a-5p, hsa-miR-1238, hsa-miR-181a-2*, hsa-miR-187, hsa-miR-30c, hsa-miR-27a, hsa-miR-99a, hsa-miR-378d, hsa-miR-181d, hsa-miR-98, hsa-miR-4685-3p, hsa-miR-598, hsa-miR-23b*, hsa-miR-3201, hsa-miR-4423-3p, hsa-miR-223, hsa-miR-3128, hsa-miR-155, hsa-miR-3163, hsa-miR-146b-5p, hsa-miR-606, hsa-miR-150, hsa-miR-4776-5p, hsa-miR-142-3p, hsa-miR-21, hsa-miR-3124-5p, hsa-miR-4741, hsa-miR-635, hsa-miR-4668-5p, hsa-miR-4763-3p, hsa-miR-4417, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

d) hsa-miR-3201, hsa-miR-4423-3p, hsa-miR-3128, hsa-miR-3163, hsa-miR-606, hsa-miR-4776-5p, hsa-miR-635, hsa-miR-3124-5p, hsa-miR-4741, hsa-miR-4668-5p, hsa-miR-4763-3p, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p.

Selon un aspect particulier, l'expression d'un ou plusieurs microARNs est en outre déterminée, lesdits ARNs étant choisis dans un des groupes consistant en hsa-miR-133a, hsa-miR-133b, hsa-miR-1, hsa-miR-486-3p, hsa-miR-34c-3p, hsa-miR-375, et hsa-miR-146b-5p.

Selon un aspect particulier, l'expression d'un ou plusieurs microARNs est en outre déterminée, lesdits ARNs, dont une augmentation de l'expression dans l'échantillon par rapport aux contrôles indique que le sujet souffre ou est susceptible de souffrir de rosacée, étant choisis dans un des groupes consistant en

1) hsa-miR-4758-5p, hsa-miR-4665-5p, hsa-miR-149*, hsa-miR-548ac, hsa-miR-4507, hsa-miR-548a-3p, hsa-miR-4289, hsa-miR-4727-3p, hsa-miR-4468, hsa-miR-4463, hsa-miR-2861, hsa-miR-4773, hsa-miR-1825, hsa-miR-4651, hsa-miR-216b, hsa-miR-4689, hsa-miR-3152-3p, hsa-miR-4270, hsa-miR-1915, hsa-miR-4739, hsa-miR-4772-5p, hsa-miR-4687-3p, hsa-miR-4674, hsa-miR-4695-5p, hsa-miR-4439, hsa-miR-129-3p, hsa-miR-1469, hsa-miR-4659a-3p, hsa-miR-4799-3p, hsa-miR-4657, hsa-miR-4655-5p, hsa-miR-4745-5p, hsa-miR-4662b, hsa-miR-4707-5p, hsa-miR-3185, hsa-miR-4260, hsa-miR-4530, hsa-miR-4734, hsa-miR-1911*, hsa-miR-4417, hsa-miR-4763-3p, hsa-miR-4668-5p, hsa-miR-1281, hsa-miR-635, hsa-miR-4741, hsa-miR-3124-5p, hsa-miR-146b-3p, hsa-miR-4529-3p, hsa-miR-4776-5p, hsa-miR-150, hsa-miR-3927, hsa-miR-606, hsa-miR-146b-5p, hsa-miR-3163, hsa-miR-155, hsa-miR-371b-5p, hsa-miR-3128, hsa-miR-4423-3p, hsa-miR-335, hsa-miR-3201, hsa-miR-184

2) hsa-miR-1269b, hsa-miR-4741, hsa-miR-4763-3p, hsa-miR-4704-5p, hsa-miR-1911*, hsa-miR-3621, hsa-miR-3612, hsa-miR-4436b-5p, hsa-miR-4734, hsa-miR-3185, hsa-miR-3910, hsa-miR-4727-3p, hsa-miR-155, hsa-miR-155*, hsa-miR-4529-3p, hsa-miR-3927, hsa-miR-3201, hsa-miR-4423-3p, hsa-miR-124, hsa-miR-184;

3) hsa-miR-4776-5p, hsa-miR-601, hsa-miR-4530, hsa-miR-4773, hsa-miR-4717-3p, hsa-miR-4657, hsa-miR-4289, hsa-miR-4417, hsa-miR-4445*, hsa-miR-150, hsa-miR-146b-5p, hsa-miR-3175, hsa-miR-4646-5p, hsa-miR-3163, hsa-miR-4423-3p, hsa-miR-3128, hsa-miR-1244, hsa-miR-21*, hsa-miR-3201, hsa-miR-335

4) hsa-miR-223, hsa-miR-142-3p, hsa-miR-146b, hsa-miR-21, hsa-miR-155

a) hsa-miR-3201, hsa-miR-4423-3p, hsa-miR-3128, hsa-miR-155, hsa-miR-3163, hsa-miR-146b-5p, hsa-miR-606, hsa-miR-150, hsa-miR-4776-5p, hsa-miR-3124-5p, hsa-miR-4741, hsa-miR-635, hsa-miR-4668-5p, hsa-miR-4763-3p, hsa-miR-4417, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

b) hsa-miR-223, hsa-miR-142-3p, hsa-miR-146b, hsa-miR-21

c) hsa-miR-3201, hsa-miR-4423-3p, hsa-miR-223, hsa-miR-3128, hsa-miR-155, hsa-miR-3163, hsa-miR-146b-5p, hsa-miR-606, hsa-miR-150, hsa-miR-4776-5p, hsa-miR-142-3p, hsa-miR-21, hsa-miR-3124-5p, hsa-miR-4741, hsa-miR-635, hsa-miR-4668-5p, hsa-miR-4763-3p, hsa-miR-4417, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

d) hsa-miR-3201, hsa-miR-4423-3p, hsa-miR-3128, hsa-miR-3163, hsa-miR-606, hsa-miR-4776-5p, hsa-miR-635, hsa-miR-3124-5p, hsa-miR-4741, hsa-miR-4668-5p, hsa-miR-4763-3p, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p, hsa-miR-146b-5p

Selon encore un autre aspect particulier, l'expression d'un ou plusieurs microARNs est en outre déterminée, lesdits ARNs, dont une diminution de l'expression dans l'échantillon par rapport aux contrôles indique que le sujet souffre ou est susceptible de souffrir de rosacée, étant choisis dans un des groupes consistant en

1) hsa-miR-133a, hsa-miR-206, hsa-miR-133b, hsa-miR-1, hsa-miR-299-5p, hsa-miR-486-3p, hsa-miR-381, hsa-miR-4324, hsa-miR-154, hsa-miR-1247, hsa-miR-1287, hsa-miR-376c, hsa-miR-195*, hsa-miR-411, hsa-miR-4269, hsa-miR-328, hsa-miR-1296, hsa-miR-34c-3p, hsa-miR-204, hsa-miR-504, hsa-miR-30c-1*, hsa-miR-615-3p, hsa-miR-654-3p, hsa-miR-505, hsa-miR-508-5p, hsa-miR-409-5p, hsa-miR-338-5p, hsa-miR-433, hsa-miR-375, hsa-miR-935, hsa-let-7d*, hsa-miR-99a*, hsa-miR-128, hsa-miR-1290, hsa-miR-331-5p, hsa-miR-4730, hsa-miR-29c*, hsa-miR-455-5p, hsa-miR-377*, hsa-miR-378e, hsa-miR-584, hsa-miR-96, hsa-miR-30a*, hsa-miR-143*, hsa-miR-370, hsa-miR-148a*, hsa-miR-489, hsa-miR-506, hsa-let-7e*, hsa-miR-30e*, hsa-miR-4787-3p, hsa-let-7b*, hsa-miR-181c*, hsa-miR-513a-5p, hsa-miR-92a-1*, hsa-miR-337-5p, hsa-miR-1181, hsa-miR-1237, hsa-miR-3620, hsa-miR-485-5p, hsa-miR-23c, hsa-miR-18a*, hsa-miR-10a, hsa-miR-139-3p, hsa-miR-493, hsa-miR-148b, hsa-miR-550a, hsa-miR-3147, hsa-miR-378g, hsa-miR-675*, hsa-miR-1238, hsa-miR-542-5p, hsa-miR-30c-2*, hsa-miR-125a-3p, hsa-miR-181a-2*, hsa-miR-1292, hsa-miR-187, hsa-miR-378*, hsa-miR-493*, hsa-miR-495, hsa-miR-557, hsa-miR-3909, hsa-miR-378d, hsa-miR-491-5p, hsa-miR-181d, hsa-miR-671-3p, hsa-miR-513c, hsa-miR-487b, hsa-miR-378b, hsa-miR-885-5p, hsa-miR-98, hsa-miR-29b-2*, hsa-miR-4685-3p, hsa-miR-3605-3p, hsa-miR-24-1*, hsa-miR-4649-3p, hsa-miR-3180-5p, hsa-miR-149, hsa-miR-23b*

2) hsa-miR-206, hsa-miR-133b, hsa-miR-133a, hsa-miR-1, hsa-miR-486-3p, hsa-miR-299-5p, hsa-miR-1247, hsa-miR-381, hsa-miR-154, hsa-miR-433, hsa-miR-1244, hsa-miR-4485, hsa-miR-378e, hsa-miR-505, hsa-miR-376c, hsa-miR-1296, hsa-miR-29b-1*, hsa-miR-409-5p, hsa-miR-29c*, hsa-miR-34c-3p, hsa-miR-4646-5p, hsa-miR-99a*, hsa-miR-378g, hsa-miR-504, hsa-miR-584, hsa-miR-128, hsa-miR-30c-1*, hsa-miR-665, hsa-miR-4269, hsa-miR-493*, hsa-miR-328, hsa-miR-550a, hsa-miR-375, hsa-miR-493, hsa-miR-378d, hsa-miR-148a*, hsa-miR-615-3p, hsa-miR-431*, hsa-miR-378i, hsa-miR-422a, hsa-miR-331-5p, hsa-miR-378*, hsa-miR-148b, hsa-miR-601, hsa-miR-4288, hsa-miR-596, hsa-miR-10a, hsa-miR-200c*, hsa-miR-378b, hsa-miR-378f, hsa-miR-4257, hsa-miR-370, hsa-miR-1237, hsa-miR-3942-3p

3) hsa-miR-211, hsa-miR-29b, hsa-miR-4324, hsa-miR-143*, hsa-miR-1287, hsa-miR-4708-5p, hsa-miR-195*, hsa-miR-508-5p, hsa-miR-204, hsa-miR-96, hsa-let-7b*, hsa-miR-935, hsa-miR-675*, hsa-miR-149, hsa-miR-30a*, hsa-miR-1181, hsa-miR-506, hsa-miR-23c, hsa-miR-127-5p, hsa-miR-491-5p

4) hsa-miR-30b, hsa-miR-221, hsa-miR-141, hsa-miR-339-3p, hsa-miR-100, hsa-miR-331, hsa-miR-598, hsa-miR-24, hsa-miR-23a, hsa-miR-99a, hsa-miR-152, hsa-miR-423-5p, hsa-miR-34c, hsa-miR-27a, hsa-miR-30c, hsa-miR-199a, hsa-miR-125a-5p, mmu-miR-491, hsa-miR-489, hsa-miR-337-5p, hsa-miR-149, hsa-miR-127, hsa-miR-375, hsa-miR-101, hsa-miR-296, mmu-miR-379, hsa-miR-574-3p, hsa-miR-487b, mmu-miR-499, hsa-miR-95, hsa-miR-885-5p, hsa-miR-486-3p, hsa-miR-1, hsa-miR-133b, hsa-miR-133a.

a) hsa-miR-133a, hsa-miR-133b, hsa-miR-1, hsa-miR-299-5p, hsa-miR-486-3p, hsa-miR-381, hsa-miR-4324, hsa-miR-154, hsa-miR-1247, hsa-miR-1287, hsa-miR-376c, hsa-miR-195*, hsa-miR-4269, hsa-miR-1296, hsa-miR-34c-3p, hsa-miR-204, hsa-miR-504, hsa-miR-30c-1*, hsa-miR-615-3p, hsa-miR-505, hsa-miR-508-5p, hsa-miR-409-5p, hsa-miR-433, hsa-miR-375, hsa-miR-935, hsa-miR-128, hsa-miR-331-5p, hsa-miR-584, hsa-miR-30a*, hsa-miR-370, hsa-miR-148a*, hsa-miR-489, hsa-miR-30e*, hsa-miR-337-5p, hsa-miR-1181, hsa-miR-1237, hsa-miR-485-5p, hsa-miR-23c, hsa-miR-148b, hsa-miR-378g, hsa-miR-1238, hsa-miR-181a-2*, hsa-miR-187, hsa-miR-378d, hsa-miR-181d, hsa-miR-98, hsa-miR-4685-3p, hsa-miR-23b*

b) hsa-miR-331, hsa-miR-598, hsa-miR-24, hsa-miR-23a, hsa-miR-99a, hsa-miR-152, hsa-miR-423-5p, hsa-miR-34c, hsa-miR-27a, hsa-miR-30c, hsa-miR-125a-5p, mmu-miR-491, hsa-miR-127, hsa-miR-375, hsa-miR-101, hsa-miR-296, mmu-miR-379, hsa-miR-574-3p, hsa-miR-487b, mmu-miR-499, hsa-miR-95, hsa-miR-885-5p, hsa-miR-486-3p, hsa-miR-1, hsa-miR-133b, hsa-miR-133a

c) hsa-miR-133a, hsa-miR-133b, hsa-miR-1, hsa-miR-299-5p, hsa-miR-486-3p, hsa-miR-381, hsa-miR-4324, hsa-miR-154, hsa-miR-1247, hsa-miR-885-5p, hsa-miR-1287, hsa-miR-95, hsa-miR-376c, hsa-miR-195*, hsa-miR-4269, mmu-miR-499, hsa-miR-1296, hsa-miR-34c-3p, hsa-miR-204, hsa-miR-504, hsa-miR-30c-1*, hsa-miR-487b, hsa-miR-615-3p, hsa-miR-505, hsa-miR-508-5p, hsa-miR-409-5p, hsa-miR-433, hsa-miR-375, hsa-miR-935, hsa-miR-128, hsa-miR-331-5p, hsa-miR-584, hsa-miR-30a*, hsa-miR-370, hsa-miR-148a*, hsa-miR-489, hsa-miR-30e*, hsa-miR-337-5p, hsa-miR-1181, hsa-miR-1237, hsa-miR-485-5p, hsa-miR-23c, hsa-miR-491-5p, hsa-miR-148b, hsa-miR-378g, hsa-miR-125a-5p, hsa-miR-1238, hsa-miR-181a-2*, hsa-miR-187, hsa-miR-30c, hsa-miR-27a, hsa-miR-99a, hsa-miR-378d, hsa-miR-181d, hsa-miR-98, hsa-miR-4685-3p, hsa-miR-598, hsa-miR-23b*

5) hsa-miR-133a, hsa-miR-133b, hsa-miR-1, hsa-miR-486-3p, hsa-miR-34c-3p, hsa-miR-375.

La détection ou la quantification du ou des échantillons biologiques du ou des sujets contrôles peuvent être concomitantes à celles effectuées pour l'échantillon du patient ou provenir de données collectées antérieurement et disponibles, par exemple, sur une base de données.

Dans les méthodes selon l'invention, l'expression des microARNs peut être détectée ou quantifiée selon des méthodes bien connues de l'homme du métier, par exemple par RT-PCR quantitative et/ou par des techniques d'hybridation, par exemple à l'aide d'une sonde marquée ou par l'utilisation d'une puce.

Un autre objet de l'invention concerne une méthode pour suivre ou déterminer l'efficacité d'un traitement de la rosacée chez un sujet comprenant la détermination dans un échantillon dudit sujet de l'expression d'un ou plusieurs microARNs choisis dans un des groupes consistant en hsa-miR-3201, hsa-miR-4423-3p, hsa-miR-3128, hsa-miR-3163, hsa-miR-606, hsa-miR-4776-5p, et hsa-miR-635, la diminution de l'expression d'un ou plusieurs microARNs à l'issue ou pendant le traitement indiquant l'efficacité du traitement.

*Applications thérapeutiques*

Il est également possible de cibler la régulation de ces microARNs pour traiter ou prévenir la rosacée; la molécule utilisée pour le traitement est une molécule permettant de diminuer ou de supprimer la dérégulation de l'expression d'un ou plusieurs microARNs différentiellement exprimés chez les sujets souffrant de rosacée. Notamment, les microARNs sous-exprimés chez les sujets souffrant de rosacée pourraient être utilisés pour le traitement de la rosacée par administration de ceux-ci. Alternativement, lorsque des microARNs sont sur-exprimés chez les sujets souffrant de rosacée, le traitement visera à permettre l'augmentation de l'expression du gène cible, par exemple en bloquant l'effet de ces microARNs.

Il est décrit ici une composition pharmaceutique comprenant un ou plusieurs microARNs identifiés et son utilisation pour le traitement de la rosacée ou la préparation d'un médicament destiné au traitement de la rosacée. De préférence, la composition comprend en outre un ou plusieurs excipients et/ou véhicules pharmaceutiquement acceptables. Il est également décrit un vecteur d'expression comprenant une séquence nucléotidique codant pour un ou plusieurs des microARNs identifiés ou un précurseurs de ceux-ci et son utilisation pour le traitement de la rosacée ou la préparation d'un médicament destiné au traitement de la rosacée. De préférence, les microARNs identifiés sont choisis parmi les microARNs sous-exprimés chez les patients souffrant de la rosacée.

Le ou les microARNs d'intérêt sont tels que décrits ici.

Il est proposé d'utiliser dans le traitement de la rosacée une molécule ou combinaison de molécules, qui peut augmenter l'expression d'un ou plusieurs des microARNs décrits ici, ou précurseurs de ces microARNs, ou un acide nucléique codant pour ledit ou lesdits microARNs ou précurseurs de ceux-ci, ou un analogue, dérivé ou forme modifiée du ou des microARNs conservant son ou leur activité.

Ladite molécule ou combinaison de molécules peut à l'inverse être un inhibiteur du ou des microARNs, de préférence un oligonucléotide sens ou antisens capable de s'hybrider au(x)dit(s) microARNs, inhibant ainsi la production et/ou l'activité du ou des microARNs ou augmentant la déplétion du ou des microARNs.

Il est également envisagé d'effectuer un criblage pour identifier de nouveaux médicaments pour le traitement de la rosacée, dans lequel les molécules candidates seront testées quant à leur capacité de restaurer totalement ou partiellement une expression des microARNs dérégulés chez les sujets souffrant de la rosacée et les molécules ayant l'effet souhaité seront sélectionnées.

Il est donc décrit ici une méthode de criblage de molécules utiles pour le traitement de la rosacée, de préférence la rosacée de type II, comprenant a) la mise en contact d'une cellule avec une molécule test, b) la détermination de l'expression des sept microARNs suivants : hsa-miR-3201, hsa-miR-4423-3p, hsa-miR-3128, hsa-miR-3163, hsa-miR-606, hsa-miR-4776-5p, et hsa-miR-635, ou capable de moduler l'expression d'un ou desdits microARNs, et c) la sélection de la molécule test si elle augmente ou diminue l'expression ou l'activité d'un ou plusieurs desdits microARNs.

Il est également décrit ici l'utilisation de molécule ou combinaison de molécules dans le traitement de la rosacée, de préférence la rosacée de type II, ladite molécule ou combinaison de molécules permettant de diminuer l'expression d'au moins un des sept microARNs suivants : hsa-miR-3201, hsa-miR-4423-3p, hsa-miR-3128, hsa-miR-3163, hsa-miR-606, hsa-miR-4776-5p, et hsa-miR-635, ou capable de moduler l'expression d'un ou des microARNs décrits ici.

Typiquement l'expression ou activité d'un microARN est analysée et comparée en présence et en absence de la molécule test.

La partie expérimentale suivante illustre l'invention sans en limiter la portée.

**EXEMPLES**

***Introduction***

Il s'agit d'une étude clinique dont les échantillons, des biopsies de peau de 3 mm prélevées au niveau du pli nasal, proviennent de trois groupes d'individus :

- 7 sujets volontaires sains (HV) pris comme témoins pour établir un différentiel avec la pathologie,
- 8 sujets atteints de rosacée ETR (RI),

- 9 sujets atteints de rosacée PPR (RII).

Les inventeurs se sont intéressés à caractériser le miRNome dans la Rosacée érythémato-télangiectasique (sous-type I) et papulopustuleuse (sous-type II).

Dans ce but, la technologie Affymetrix a été utilisée, rendant possible l'étude à large échelle des microARNs .

Concernant les analyses statistiques, les données d'expression à large échelle ont été filtrées sur l'espèce *Homo sapiens* puis normalisées par RMA (Robust Multiarray/Multichip Average). Un filtre sur les niveaux d'expression a ensuite été appliqué. Par la suite, les données normalisées ont été statistiquement comparées par analyse différentielle afin d'établir une liste de microARNs significativement modulés de façon spécifique dans la peau lésionnelle (sujets malades) par rapport à de la peau saine (sujets sains). Enfin, l'interprétation biologique a été permise à partir d'une liste de microARNs modulés dans la rosacée papulopustuleuse grâce au logiciel Ingenuity Pathway Analysis, en interconnectant les données issues de l'étude transcriptomique (profils d'expression des ARNm) des sujets.

La caractérisation du miRNome de la pathologie passe dans un premier temps par une étude à large échelle, à l'aide des puces Affymetrix miRNA 3.0, afin d'établir les profils d'expression des microARNs. Une analyse approfondie a permis d'établir la méthode de travail à adopter : filtration des niveaux d'expression, normalisation des données puis analyse différentielle.

Pour compléter les données issues du miRNome, les données de transcriptomique (profils d'Expression des ARNm) issues de cette même étude ont été associées.

Bien que tous les échantillons aient été traités, peu de microARNs d'intérêt se sont avérés différentiellement exprimés dans RI par rapport à la peau saine. De ce fait, le projet s'est focalisé sur l'étude de microARNs permettant de discriminer des sujets malades atteints d'une Rosacée de type II (papulopustuleuse) par rapport à des sujets ne présentant pas de Rosacée. Une liste de, 92 microARNs ont été mis en évidence comme étant différentiellement exprimés de manière significative RII vs HV.

Dans le but de rechercher d'éventuels microARNs biomarqueurs, l'expression des microARNs a été comparée dans 3 pathologies cutanées : le Psoriasis, l'Acné et la Rosacée. De façon intéressante, nous avons observé une majorité de microARNs modulés de façon spécifique dans chaque pathologie, confirmant les signatures caractéristiques de ces petits ARNs régulateurs. Dans ce projet, 7 microARNs biomarqueurs de la Rosacée ont été mis en évidence.

### *Résultats*

Etude à large échelle des microARNs

*a) Contrôles qualité de la puce Affymetrix miRNA 3.0*

Après normalisation RMA des données expérimentales, une série de contrôles qualité sont réalisés.

Parmi eux on trouve :

- Le contrôle du bruit de fond moyen avec $\frac{valeur\ \max bruit}{valeur\ \min bruit} < 3$ pour les 24 échantillons.
- Les 5 oligonucléotides contrôles injectés dans les échantillons d'ARN avant l'étape de biotinylation des ARN montrent une intensité moyenne supérieure à 10; ces contrôles valident les étapes de polyadénylation et de ligation.
- Les 4 cibles injectées au moment de l'étape d'hybridation des ARN sur les sondes de la puce respectent le profil attendu : $Intensité_{bioB} < Intensité_{bioC} < Intensité_{bioD} < Intensité_{Cre}$ ; ces contrôles attestent du bon déroulement de l'étape d'hybridation.

Enfin, l'analyse en composantes principales permet de détecter d'éventuels échantillons aberrants et d'observer d'éventuels regroupements d'échantillons de façon très globale. Aucun échantillon ne sort du cercle de confiance. De ce fait, nous avons pu conserver tous les échantillons de l'étude.

*b) Normalisation RMA (Robust MultiArray Average)*

La normalisation RMA a été réalisée :

- A partir de tous les ensembles de cibles présents sur la puce soit 25 119 identifiants.
- A partir de tous les ensembles de cibles humains soit 5683 identifiants. La normalisation s'est alors faite au moyen d'expression console en filtrant les données d'expression sur l'espèce *Homo sapiens.*

Ces données normalisées ont ensuite été comparées au moyen d'une analyse différentielle comparant les individus RII aux individus sains. Comme ces deux analyses ont montré de très faibles différences, les analyses suivantes ont été réalisées à partir des données normalisées dans Expression Console sur l'humain (soit 5683 identifiants). Par ailleurs, les analyses différentielles RI versus HV et RII versus RI ont révélé moins de microARNs modulés, c'est pourquoi les analyses suivantes se sont focalisées sur l'analyse différentielle du miRNome des individus malades RII versus le miRNome des individus sains HV avec 29 microARNs significativement modulés sans filtre sur le niveau d'expression.

**Tableau 1** :

|  | RI vs HV | RII vs HV | RII vs RI |
|---|---|---|---|
| Nombre de microARNs modulés* avec \|Fold\|≥1,5 et p-value≤0,05 | 74 | 160 | 40 |
| Nombre de microARNs modulés** avec \|Fold\|≥1,5 et FDR≤0,05 | 1 | 29 | 1 |
| *microARNs humains matures avec \|Fold\|≥ 1,5 et p-value ≤ 0,05 (sans correction par le FDR) et sans filtre sur le niveau d'expression avant analyse différentielle.<br>**microARNs humains matures avec \|Fold\|≥ 1,5 et FDR ≤ 0,05 sans filtre sur le niveau d'expression avant analyse différentielle. | | | |

**Tableau 2 : Liste des 74 microARNs matures modulés (\|fold\|≥1,5 et raw p-value≤0.05) dans l'analyse différentielle RI vs HV (étude Rosacée Blume : GRDS0050) à partir des 3391 identifiants Affymetrix**

| Affymetrix id | FoldChange | RawPValue | FDR_BH | mean expression RI | mean expression HV |
|---|---|---|---|---|---|
| hsa-miR-206 | -36,5 | 2,4E-02 | 4,6E-01 | 33 | 1213 |
| hsa-miR-133b | -24,8 | 7,9E-03 | 3,5E-01 | 19 | 466 |
| hsa-miR-133a | -23,6 | 2,4E-02 | 4,6E-01 | 24 | 562 |
| hsa-miR-1 | -6,2 | 9,0E-03 | 3,5E-01 | 5 | 31 |
| hsa-miR-486-3p | -3,9 | 1,3E-02 | 3,9E-01 | 12 | 48 |
| hsa-miR-299-5p | -3,2 | 2,6E-02 | 4,8E-01 | 11 | 36 |
| hsa-miR-1247 | -3,0 | 1,1E-03 | 2,0E-01 | 10 | 29 |
| hsa-miR-381 | -2,9 | 2,2E-02 | 4,6E-01 | 18 | 52 |
| hsa-miR-154 | -2,9 | 2,4E-05 | 4,1E-02 | 10 | 28 |
| hsa-miR-433 | -2,4 | 1,7E-03 | 2,0E-01 | 6 | 14 |
| hsa-miR-1244 | -2,4 | 7,1E-03 | 3,3E-01 | 7 | 15 |
| hsa-miR-4485 | -2,3 | 5,8E-03 | 3,2E-01 | 21 | 48 |
| hsa-miR-378e | -2,3 | 2,0E-03 | 2,0E-01 | 48 | 109 |
| hsa-miR-505 | -2,2 | 8,5E-03 | 3,5E-01 | 12 | 26 |
| hsa-miR-376c | -2,2 | 3,1E-02 | 4,9E-01 | 5 | 11 |
| hsa-miR-1296 | -2,2 | 1,1E-03 | 2,0E-01 | 8 | 17 |
| hsa-miR-29b-1* | -2,2 | 3,0E-02 | 4,9E-01 | 15 | 33 |
| hsa-miR-409-5p | -2,1 | 1,5E-02 | 4,2E-01 | 11 | 22 |
| hsa-miR-29c* | -2,1 | 1,2E-02 | 3,8E-01 | 12 | 25 |
| hsa-miR-34c-3p | -2,1 | 6,0E-04 | 1,5E-01 | 7 | 13 |
| hsa-miR-4646-5p | -2,0 | 6,0E-04 | 1,5E-01 | 7 | 14 |
| hsa-miR-99a* | -2,0 | 1,7E-02 | 4,2E-01 | 14 | 28 |
| hsa-miR-378g | -2,0 | 1,0E-04 | 1,2E-01 | 144 | 281 |
| hsa-miR-504 | -1,9 | 2,1E-02 | 4,5E-01 | 8 | 16 |
| hsa-miR-584 | -1,9 | 5,9E-03 | 3,2E-01 | 13 | 24 |
| hsa-miR-128 | -1,9 | 8,0E-03 | 3,5E-01 | 52 | 98 |
| hsa-miR-30c-1* | -1,8 | 9,3E-03 | 3,5E-01 | 9 | 17 |
| hsa-miR-665 | -1,8 | 3,1E-02 | 4,9E-01 | 8 | 15 |
| hsa-miR-4269 | -1,8 | 4,0E-03 | 2,9E-01 | 26 | 46 |
| hsa-miR-493* | -1,8 | 8,0E-04 | 1,8E-01 | 2 | 4 |
| hsa-miR-328 | -1,8 | 8,8E-03 | 3,5E-01 | 16 | 28 |
| hsa-miR-550a | -1,8 | 2,4E-02 | 4,6E-01 | 6 | 10 |

(suite)

| Affymetrix id | FoldChange | RawPValue | FDR_BH | mean expression RI | mean expression HV |
|---|---|---|---|---|---|
| hsa-miR-375 | -1,7 | **1,6E-02** | 4,2E-01 | 29 | 50 |
| hsa-miR-493 | -1,7 | **1,9E-02** | 4,3E-01 | 3 | 5 |
| hsa-miR-378d | -1,7 | **1,0E-4** | 1,2E-01 | 270 | 464 |
| hsa-miR-148a* | -1,7 | **1,4E-02** | 4,1E-01 | 5 | 9 |
| hsa-miR-615-3p | -1,7 | **7,0E-03** | 3,3E-01 | 3 | 5 |
| hsa-miR-431* | -1,7 | **2,0E-03** | 2,0E-01 | 2 | 4 |
| hsa-miR-378i | -1,7 | **4,0E-04** | 1,3E-01 | 483 | 817 |
| hsa-miR-422a | -1,7 | **3,0E-04** | 1,3E-01 | 480 | 810 |
| hsa-miR-331-5p | -1,7 | **5,0E-02** | 5,5E-01 | 7 | 12 |
| hsa-miR-378* | -1,7 | **9,2E-03** | 3,5E-01 | 161 | 267 |
| hsa-miR-148b | -1,7 | **1,6E-03** | 2,0E-01 | 39 | 64 |
| hsa-miR-601 | -1,6 | **2,0E-04** | 1,3E-01 | 2 | 3 |
| hsa-miR-4288 | -1,6 | **3,5E-03** | 2,8E-01 | 4 | 6 |
| hsa-miR-596 | -1,6 | **4,9E-02** | 5,5E-01 | 4 | 6 |
| hsa-miR-10a | -1,6 | **4,7E-02** | 5,5E-01 | 5 | 8 |
| hsa-miR-200c* | -1,6 | **5,0E-02** | 5,5E-01 | 8 | 13 |
| hsa-miR-378b | -1,6 | **1,1E-02** | 3,7E-01 | 15 | 23 |
| hsa-miR-378f | -1,6 | **1,2E-03** | 2,0E-01 | 641 | 1002 |
| hsa-miR-4257 | -1,5 | **1,7E-02** | 4,2E-01 | 3 | 4 |
| hsa-miR-370 | -1,5 | **9,6E-03** | 3,5E-01 | 15 | 23 |
| hsa-miR-1237 | -1,5 | **2,7E-02** | 4,8E-01 | 3 | 5 |
| hsa-miR-3942-3p | 1,5 | **1,5E-02** | 4,2E-01 | 4 | 3 |
| hsa-miR-1269b | 1,5 | **2,3E-03** | 2,1E-01 | 3 | 2 |
| hsa-miR-4741 | 1,5 | **1,9E-02** | 4,3E-01 | 439 | 288 |
| hsa-miR-4763-3p | 1,5 | **1,8E-02** | 4,3E-01 | 1889 | 1235 |
| hsa-miR-4704-5p | 1,5 | **3,6E-02** | 5,1E-01 | 6 | 4 |
| hsa-miR-1911* | 1,5 | **3,5E-02** | 5,1E-01 | 4 | 3 |
| hsa-miR-3621 | 1,5 | **4,1E-02** | 5,3E-01 | 809 | 525 |
| hsa-miR-3612 | 1,5 | **5,4E-03** | 3,2E-01 | 5 | 3 |
| hsa-miR-4436b-5p | 1,5 | **4,3E-02** | 5,4E-01 | 67 | 44 |
| hsa-miR-4734 | 1,6 | **1,3E-02** | 4,1E-01 | 1326 | 855 |
| hsa-miR-3185 | 1,6 | **1,8E-02** | 4,3E-01 | 743 | 479 |
| hsa-miR-3910 | 1,7 | **6,3E-03** | 3,3E-01 | 16 | 10 |
| hsa-miR-4727-3p | 1,7 | **1,9E-03** | 2,0E-01 | 13 | 8 |
| hsa-miR-155 | 1,7 | **4,6E-02** | 5,5E-01 | 683 | 397 |
| hsa-miR-155* | 1,8 | **1,8E-02** | 4,3E-01 | 8 | 5 |
| hsa-miR-4529-3p | 1,9 | **4,7E-02** | 5,5E-01 | 57 | 30 |
| hsa-miR-3927 | 1,9 | **2,8E-02** | 4,9E-01 | 10 | 5 |
| hsa-miR-3201 | 2,0 | **1,6E-02** | 4,2E-01 | 102 | 51 |
| hsa-miR-4423-3p | 2,3 | **3,9E-03** | 2,9E-01 | 20 | 9 |
| hsa-miR-124 | 6,7 | **2,1E-03** | 2,0E-01 | 28 | 4 |
| hsa-miR-184 | 15,9 | **9,0E-04** | 1,9E-01 | 39 | 2 |

**Tableau 3 : Liste des 160 microARNs matures modulés (|fold|≥1,5 et raw p-value≤0.05) dans l'analyse différentielle RII vs HV (étude Rosacée Blume : GRDS0050) à partir des 3391 identifiants Affymetrix**

| Affymetrix id | Fold Change | RawPValue | FDR_BH | mean expression HV | mean expression RII |
|---|---|---|---|---|---|
| hsa-miR-133a | -45,81 | **6,4E-03** | 1,2E-01 | 562 | 12 |
| hsa-miR-206 | -43,52 | **1,6E-02** | 1,9E-01 | 1213 | 28 |
| hsa-miR-133b | -38,97 | **2,4E-03** | 8,1E-02 | 466 | 12 |
| hsa-miR-1 | -9,71 | **1,4E-03** | 6,7E-02 | 31 | 3 |
| hsa-miR-299-5p | -5,11 | **2,5E-03** | 8,1E-02 | 36 | 7 |
| hsa-miR-486-3p | -4,67 | **4,8E-03** | 1,1E-01 | 48 | 10 |
| hsa-miR-381 | -3,54 | **6,5E-03** | 1,2E-01 | 52 | 15 |
| hsa-miR-4324 | -3,53 | **7,0E-04** | 4,4E-02 | 20 | 6 |
| hsa-miR-154 | -3,49 | **1,6E-06** | 2,3E-03 | 28 | 8 |
| hsa-miR-1247 | -3,44 | **3,0E-04** | 2,6E-02 | 29 | 9 |
| hsa-miR-1287 | -3,28 | **8,0E-04** | 4,8E-02 | 14 | 4 |
| hsa-miR-376c | -2,81 | **5,7E-03** | 1,1E-01 | 11 | 4 |
| hsa-miR-195* | -2,78 | **5,0E-04** | 3,7E-02 | 16 | 6 |
| hsa-miR-411 | -2,74 | **2,7E-02** | 2,4E-01 | 19 | 7 |
| hsa-miR-4269 | -2,63 | **1,5E-05** | 3,4E-03 | 46 | 18 |
| hsa-miR-328 | -2,59 | **6,0E-05** | 9,7E-03 | 28 | 11 |
| hsa-miR-1296 | -2,53 | **2,0E-04** | 1,8E-02 | 17 | 7 |
| hsa-miR-34c-3p | -2,53 | **2,5E-05** | 5,2E-03 | 13 | 5 |
| hsa-miR-204 | -2,50 | **1,1E-03** | 5,8E-02 | 9 | 4 |
| hsa-miR-504 | -2,48 | **2,1E-03** | 7,4E-02 | 16 | 6 |
| hsa-miR-30c-1* | -2,47 | **2,0E-04** | 2,3E-02 | 17 | 7 |
| hsa-miR-615-3p | -2,41 | **4,7E-05** | 8,3E-03 | 5 | 2 |
| hsa-miR-654-3p | -2,37 | **7,9E-03** | 1,3E-01 | 16 | 7 |
| hsa-miR-505 | -2,34 | **4,6E-03** | 1,1E-01 | 26 | 11 |
| hsa-miR-508-5p | -2,33 | **1,0E-04** | 1,6E-02 | 13 | 6 |
| hsa-miR-409-5p | -2,31 | **5,4E-03** | 1,1E-01 | 22 | 10 |
| hsa-miR-338-5p | -2,25 | **3,2E-02** | 2,5E-01 | 14 | 6 |
| hsa-miR-433 | -2,25 | **2,9E-03** | 8,9E-02 | 14 | 6 |
| hsa-miR-375 | -2,22 | **1,0E-03** | 5,2E-02 | 50 | 23 |
| hsa-miR-935 | -2,19 | **8,0E-04** | 4,8E-02 | 9 | 4 |
| hsa-let-7d* | -2,15 | **1,2E-02** | 1,7E-01 | 14 | 7 |
| hsa-miR-99a* | -2,13 | **8,9E-03** | 1,4E-01 | 28 | 13 |
| hsa-miR-128 | -2,13 | **1,8E-03** | 7,2E-02 | 98 | 46 |
| hsa-miR-1290 | -2,11 | **1,9E-02** | 2,0E-01 | 104 | 49 |
| hsa-miR-331-5p | -2,11 | **5,6E-03** | 1,1E-01 | 12 | 6 |
| hsa-miR-4730 | -2,10 | **2,0E-02** | 2,1E-01 | 15 | 7 |
| hsa-miR-29c* | -2,08 | **9,9E-03** | 1,5E-01 | 25 | 12 |
| hsa-miR-455-5p | -2,04 | **1,7E-02** | 2,0E-01 | 12 | 6 |
| hsa-miR-377* | -1,98 | **4,2E-02** | 3,0E-01 | 7 | 4 |
| hsa-miR-378e | -1,98 | **7,2E-03** | 1,3E-01 | 109 | 55 |
| hsa-miR-584 | -1,96 | **3,9E-03** | 1,0E-01 | 24 | 12 |
| hsa-miR-96 | -1,93 | **1,1E-02** | 1,6E-01 | 6 | 3 |
| hsa-miR-30a* | -1,92 | **1,1E-05** | 3,3E-03 | 164 | 85 |
| hsa-miR-143* | -1,92 | **4,2E-02** | 3,0E-01 | 15 | 8 |
| hsa-miR-370 | -1,91 | **2,0E-04** | 1,8E-02 | 23 | 12 |
| hsa-miR-148a* | -1,88 | **3,4E-03** | 9,3E-02 | 9 | 5 |
| hsa-miR-489 | -1,86 | **5,2E-03** | 1,1E-01 | 53 | 29 |
| hsa-miR-506 | -1,85 | **1,1E-02** | 1,6E-01 | 5 | 3 |

(suite)

| Affymetrix id | Fold Change | RawPValue | FDR_BH | mean expression HV | mean expression RII |
|---|---|---|---|---|---|
| hsa-let-7e* | -1,84 | **1,6E-03** | 6,7E-02 | 4 | 2 |
| hsa-miR-30e* | -1,83 | **5,0E-03** | 1,1E-01 | 62 | 34 |
| hsa-miR-4787-3p | -1,81 | **1,3E-02** | 1,7E-01 | 11 | 6 |
| hsa-let-7b* | -1,80 | **7,2E-03** | 1,3E-01 | 11 | 6 |
| hsa-miR-181c* | -1,80 | **9,6E-03** | 1,5E-01 | 8 | 4 |
| hsa-miR-513a-5p | -1,79 | **4,0E-02** | 2,9E-01 | 28 | 15 |
| hsa-miR-92a-1* | -1,79 | **2,4E-02** | 2,3E-01 | 30 | 17 |
| hsa-miR-337-5p | -1,79 | **2,3E-03** | 7,9E-02 | 35 | 19 |
| hsa-miR-1181 | -1,77 | **5,9E-03** | 1,2E-01 | 12 | 7 |
| hsa-miR-1237 | -1,75 | **3,3E-03** | 9,1E-02 | 5 | 3 |
| hsa-miR-3620 | -1,75 | **2,8E-02** | 2,4E-01 | 8 | 5 |
| hsa-miR-485-5p | -1,74 | **1,9E-03** | 7,4E-02 | 23 | 13 |
| hsa-miR-23c | -1,74 | **4,3E-03** | 1,0E-01 | 79 | 45 |
| hsa-miR-18a* | -1,74 | **3,3E-02** | 2,6E-01 | 23 | 13 |
| hsa-miR-10a | -1,74 | **1,9E-02** | 2,0E-01 | 8 | 5 |
| hsa-miR-139-3p | -1,73 | **1,2E-02** | 1,7E-01 | 31 | 18 |
| hsa-miR-493 | -1,72 | **1,8E-02** | 2,0E-01 | 5 | 3 |
| hsa-miR-148b | -1,71 | **7,0E-04** | **4,6E-02** | 64 | 37 |
| hsa-miR-550a | -1,70 | **2,7E-02** | 2,4E-01 | 10 | 6 |
| hsa-miR-3147 | -1,70 | **1,2E-02** | 1,7E-01 | 16 | 9 |
| hsa-miR-378g | -1,69 | **1,2E-03** | 6,2E-02 | 281 | 166 |
| hsa-miR-675* | -1,66 | **2,5E-02** | 2,3E-01 | 6 | 4 |
| hsa-miR-1238 | -1,66 | **6,0E-03** | 1,2E-01 | 10 | 6 |
| hsa-miR-542-5p | -1,66 | **3,4E-02** | 2,6E-01 | 8 | 5 |
| hsa-miR-30c-2* | -1,65 | **8,6E-03** | 1,4E-01 | 22 | 13 |
| hsa-miR-125a-3p | -1,65 | **7,3E-03** | 1,3E-01 | 34 | 20 |
| hsa-miR-181a-2* | -1,65 | **2,9E-03** | 8,9E-02 | 155 | 94 |
| hsa-miR-1292 | -1,64 | **4,0E-02** | 2,9E-01 | 11 | 7 |
| hsa-miR-187 | -1,63 | **4,3E-03** | 1,0E-01 | 56 | 35 |
| hsa-miR-378* | -1,63 | **1,1E-02** | 1,6E-01 | 267 | 164 |
| hsa-miR-493* | -1,61 | **2,9E-03** | 8,9E-02 | 4 | 3 |
| hsa-miR-495 | -1,61 | **4,0E-02** | 2,9E-01 | 5 | 3 |
| hsa-miR-557 | -1,60 | **2,7E-02** | 2,4E-01 | 10 | 7 |
| hsa-miR-3909 | -1,59 | **2,9E-02** | 2,4E-01 | 4 | 2 |
| hsa-miR-378d | -1,58 | **6,0E-04** | **4,1E-02** | 464 | 295 |
| hsa-miR-491-5p | -1,57 | **9,3E-03** | 1,5E-01 | 38 | 24 |
| hsa-miR-181d | -1,57 | **1,7E-03** | 7,1E-02 | 73 | 47 |
| hsa-miR-671-3p | -1,57 | **3,7E-02** | 2,8E-01 | 11 | 7 |
| hsa-miR-513c | -1,56 | **4,7E-02** | 3,2E-01 | 6 | 4 |
| hsa-miR-487b | -1,56 | **1,2E-02** | 1,6E-01 | 157 | 101 |
| hsa-miR-378b | -1,56 | **1,0E-02** | 1,5E-01 | 23 | 15 |
| hsa-miR-885-5p | -1,54 | **5,0E-02** | 3,3E-01 | 41 | 26 |
| hsa-miR-98 | -1,54 | **5,2E-03** | 1,1E-01 | 74 | 48 |
| hsa-miR-29b-2* | -1,54 | **8,7E-03** | 1,4E-01 | 61 | 40 |
| hsa-miR-4685-3p | -1,53 | **5,2E-03** | 1,1E-01 | 5 | 3 |
| hsa-miR-3605-3p | -1,52 | **1,2E-02** | 1,6E-01 | 4 | 2 |
| hsa-miR-24-1* | -1,52 | **3,2E-02** | 2,5E-01 | 4 | 3 |
| hsa-miR-4649-3p | -1,51 | **1,7E-02** | 1,9E-01 | 7 | 5 |
| hsa-miR-3180-5p | -1,51 | **2,1E-03** | 7,4E-02 | 5 | 3 |

(suite)

| Affymetrix id | Fold Change | RawPValue | FDR_BH | mean expression HV | mean expression RII |
|---|---|---|---|---|---|
| hsa-miR-149 | -1,50 | **2,8E-02** | 2,4E-01 | 595 | 396 |
| hsa-miR-23b* | -1,50 | **4,9E-03** | 1,1E-01 | 56 | 37 |
| hsa-miR-4758-5p | 1,50 | **2,4E-02** | 2,3E-01 | 195 | 293 |
| hsa-miR-4665-5p | 1,50 | **1,3E-02** | 1,7E-01 | 132 | 198 |
| hsa-miR-149* | 1,50 | **3,0E-02** | 2,5E-01 | 1169 | 1759 |
| hsa-miR-548ac | 1,51 | **4,7E-02** | 3,2E-01 | 15 | 23 |
| hsa-miR-4507 | 1,51 | **1,5E-02** | 1,8E-01 | 221 | 334 |
| hsa-miR-548a-3p | 1,51 | **3,8E-03** | 1,0E-01 | 23 | 35 |
| hsa-miR-4289 | 1,52 | **2,5E-02** | 2,4E-01 | 3 | 5 |
| hsa-miR-4727-3p | 1,53 | **7,2E-03** | 1,3E-01 | 8 | 12 |
| hsa-miR-4468 | 1,54 | **8,7E-05** | **1,2E-02** | 2 | 3 |
| hsa-miR-4463 | 1,54 | **4,2E-02** | 2,9E-01 | 622 | 956 |
| hsa-miR-2861 | 1,54 | **9,8E-03** | 1,5E-01 | 3594 | 5552 |
| hsa-miR-4773 | 1,55 | **2,0E-02** | 2,1E-01 | 8 | 12 |
| hsa-miR-1825 | 1,57 | **4,6E-02** | 3,1E-01 | 53 | 84 |
| hsa-miR-4651 | 1,58 | **1,1E-02** | 1,6E-01 | 567 | 893 |
| hsa-miR-216b | 1,58 | **4,1E-03** | 1,0E-01 | 3 | 5 |
| hsa-miR-4689 | 1,59 | **3,1E-02** | 2,5E-01 | 239 | 378 |
| hsa-miR-3152-3p | 1,59 | **4,6E-02** | 3,1E-01 | 7 | 12 |
| hsa-miR-4270 | 1,60 | **8,7E-03** | 1,4E-01 | 695 | 1111 |
| hsa-miR-1915 | 1,60 | **3,0E-03** | 8,9E-02 | 3941 | 6301 |
| hsa-miR-4739 | 1,62 | **2,7E-03** | 8,5E-02 | 753 | 1221 |
| hsa-miR-4772-5p | 1,64 | **1,2E-02** | 1,7E-01 | 3 | 5 |
| hsa-miR-4687-3p | 1,65 | **2,0E-04** | **1,8E-02** | 2168 | 3569 |
| hsa-miR-4674 | 1,65 | **3,0E-03** | 8,9E-02 | 388 | 640 |
| hsa-miR-4695-5p | 1,65 | **1,0E-03** | 5,2E-02 | 325 | 537 |
| hsa-miR-4439 | 1,65 | **3,2E-02** | 2,5E-01 | 4 | 6 |
| hsa-miR-129-3p | 1,66 | **2,2E-02** | 2,2E-01 | 6 | 9 |
| hsa-miR-1469 | 1,66 | **6,0E-04** | **4,0E-02** | 2171 | 3606 |
| hsa-miR-4659a-3p | 1,66 | **5,0E-04** | **3,7E-02** | 2 | 4 |
| hsa-miR-4799-3p | 1,66 | **3,5E-02** | 2,6E-01 | 3 | 5 |
| hsa-miR-4657 | 1,68 | **7,8E-03** | 1,3E-01 | 8 | 14 |
| hsa-miR-4655-5p | 1,68 | **8,7E-03** | 1,4E-01 | 47 | 79 |
| hsa-miR-4745-5p | 1,69 | **3,0E-03** | 8,9E-02 | 1374 | 2321 |
| hsa-miR-4662b | 1,70 | **2,4E-03** | 8,0E-02 | 2 | 4 |
| hsa-miR-4707-5p | 1,70 | **4,6E-03** | 1,1E-01 | 903 | 1535 |
| hsa-miR-3185 | 1,71 | **4,4E-03** | 1,1E-01 | 479 | 816 |
| hsa-miR-4260 | 1,71 | **3,6E-03** | 9,8E-02 | 3 | 6 |
| hsa-miR-4530 | 1,74 | **4,3E-03** | 1,0E-01 | 786 | 1371 |
| hsa-miR-4734 | 1,75 | **2,0E-03** | 7,4E-02 | 855 | 1495 |
| hsa-miR-1911* | 1,76 | **5,9E-03** | 1,2E-01 | 3 | 5 |
| hsa-miR-4417 | 1,77 | **4,6E-03** | 1,1E-01 | 32 | 56 |
| hsa-miR-4763-3p | 1,77 | **1,9E-03** | 7,4E-02 | 1235 | 2182 |
| hsa-miR-4668-5p | 1,78 | **3,0E-04** | **2,5E-02** | 1388 | 2476 |
| hsa-miR-1281 | 1,82 | **1,8E-02** | 2,0E-01 | 115 | 210 |
| hsa-miR-635 | 1,85 | **1,6E-03** | 6,7E-02 | 8 | 14 |
| hsa-miR-4741 | 1,86 | **9,0E-04** | **5,0E-02** | 288 | 537 |
| hsa-miR-3124-5p | 1,89 | **2,0E-03** | 7,4E-02 | 46 | 88 |

(suite)

| Affymetrix id | Fold Change | RawPValue | FDR_BH | mean expression HV | mean expression RII |
|---|---|---|---|---|---|
| hsa-miR-146b-3p | 1,91 | **3,8E-02** | 2,8E-01 | 3 | 7 |
| hsa-miR-4529-3p | 1,93 | **3,7E-02** | 2,7E-01 | 30 | 58 |
| hsa-miR-4776-5p | 2,14 | **7,5E-06** | 3,2E-03 | 8 | 17 |
| hsa-miR-150 | 2,15 | **3,1E-03** | 9,0E-02 | 586 | 1258 |
| hsa-miR-3927 | 2,17 | **8,6E-03** | 1,4E-01 | 5 | 11 |
| hsa-miR-606 | 2,18 | **3,2E-03** | 9,1E-02 | 3 | 7 |
| hsa-miR-146b-5p | 2,32 | **3,0E-04** | 2,5E-02 | 183 | 423 |
| hsa-miR-3163 | 2,41 | **4,0E-04** | 3,0E-02 | 4 | 10 |
| hsa-miR-155 | 2,44 | **1,8E-03** | 7,2E-02 | 397 | 968 |
| hsa-miR-371b-5p | 2,48 | **7,4E-03** | 1,3E-01 | 80 | 199 |
| hsa-miR-3128 | 3,00 | **1,0E-05** | 3,3E-03 | 34 | 102 |
| hsa-miR-4423-3p | 4,35 | **7,7E-06** | 3,2E-03 | 9 | 38 |
| hsa-miR-335 | 4,42 | **1,0E-06** | 2,3E-03 | 12 | 55 |
| hsa-miR-3201 | 4,91 | **4,4E-06** | 3,2E-03 | 51 | 248 |
| hsa-miR-184 | 7,73 | **8,0E-03** | 1,3E-01 | 2 | 19 |

**Tableau 4: Liste des 40 microARNs matures modulés (|fold|≥1,5 et raw p-value≤0.05) dans l'analyse différentielle RII vs RI (étude Rosacée Blume : GRDS0050) à partir des 3391 identifiants Affymetrix**

| Affymetrix id | Fold Change | Raw PValue | FDR_BH | Mean expression RI | Mean expression RII |
|---|---|---|---|---|---|
| hsa-miR-211 | -3,3 | **4,7E-03** | 3,9E-01 | 18 | 6 |
| hsa-miR-29b | -2,8 | **4,2E-02** | 6,4E-01 | 50 | 18 |
| hsa-miR-4324 | -2,6 | **4,6E-03** | 3,9E-01 | 15 | 6 |
| hsa-miR-143* | -2,5 | **5,0E-03** | 3,9E-01 | 19 | 8 |
| hsa-miR-1287 | -2,1 | **2,2E-02** | 5,8E-01 | 9 | 4 |
| hsa-miR-4708-5p | -2,0 | **1,3E-02** | 5,0E-01 | 23 | 11 |
| hsa-miR-195* | -2,0 | **1,0E-02** | 4,9E-01 | 11 | 6 |
| hsa-miR-508-5p | -1,9 | **1,2E-03** | 2,6E-01 | 11 | 6 |
| hsa-miR-204 | -1,9 | **1,2E-02** | 5,0E-01 | 7 | 4 |
| hsa-miR-96 | -1,9 | **1,0E-02** | 4,9E-01 | 6 | 3 |
| hsa-let-7b* | -1,8 | **4,4E-03** | 3,9E-01 | 11 | 6 |
| hsa-miR-935 | -1,8 | **6,2E-03** | 4,1E-01 | 7 | 4 |
| hsa-miR-675* | -1,7 | **1,3E-02** | 5,0E-01 | 6 | 4 |
| hsa-miR-149 | -1,7 | **5,3E-03** | 3,9E-01 | 663 | 396 |
| hsa-miR-30a* | -1,6 | **2,0E-04** | 1,2E-01 | 140 | 85 |
| hsa-miR-1181 | -1,6 | **1,6E-02** | 5,3E-01 | 11 | 7 |
| hsa-miR-506 | -1,6 | **4,9E-02** | 6,5E-01 | 5 | 3 |
| hsa-miR-23c | -1,6 | **1,6E-02** | 5,2E-01 | 70 | 45 |
| hsa-miR-127-5p | -1,5 | **7,7E-03** | 4,6E-01 | 4 | 3 |
| hsa-miR-491-5p | -1,5 | **1,3E-02** | 5,0E-01 | 37 | 24 |
| hsa-miR-4776-5p | 1,5 | **3,8E-03** | 3,9E-01 | 11 | 17 |
| hsa-miR-601 | 1,5 | **8,0E-04** | 2,1E-01 | 2 | 3 |
| hsa-miR-4530 | 1,5 | **2,2E-02** | 5,8E-01 | 906 | 1371 |
| hsa-miR-4773 | 1,5 | **2,2E-02** | 5,8E-01 | 8 | 12 |
| hsa-miR-4717-3p | 1,5 | **4,7E-03** | 3,9E-01 | 6 | 10 |
| hsa-miR-4657 | 1,6 | **1,4E-02** | 5,0E-01 | 9 | 14 |
| hsa-miR-4289 | 1,6 | **8,7E-03** | 4,6E-01 | 3 | 5 |
| hsa-miR-4417 | 1,6 | **9,1E-03** | 4,7E-01 | 34 | 56 |

(suite)

| Affymetrix id | Fold Change | Raw PValue | FDR_BH | Mean expression RI | Mean expression RII |
|---|---|---|---|---|---|
| hsa-miR-4445* | 1,7 | **2,5E-02** | 6,1E-01 | 8 | 13 |
| hsa-miR-150 | 1,7 | **3,3E-02** | 6,1E-01 | 760 | 1258 |
| hsa-miR-146b-5p | 1,7 | **1,2E-02** | 5,0E-01 | 254 | 423 |
| hsa-miR-3175 | 1,8 | **1,2E-02** | 5,0E-01 | 14 | 25 |
| hsa-miR-4646-5p | 1,9 | **1,0E-03** | 2,3E-01 | 7 | 13 |
| hsa-miR-3163 | 1,9 | **5,3E-03** | 3,9E-01 | 6 | 10 |
| hsa-miR-4423-3p | 1,9 | **1,5E-02** | 5,2E-01 | 20 | 38 |
| hsa-miR-3128 | 2,2 | **4,0E-04** | 1,5E-01 | 47 | 102 |
| hsa-miR-1244 | 2,3 | **5,4E-03** | 3,9E-01 | 7 | 15 |
| hsa-miR-21* | 2,4 | **1,9E-02** | 5,8E-01 | 12 | 29 |
| hsa-miR-3201 | 2,4 | **1,8E-03** | 3,2E-01 | 102 | 248 |
| hsa-miR-335 | 3,9 | **2,5E-06** | **8,4E-03** | 14 | 55 |

*c) Filtre des données sur les niveaux d'expression*

L'analyse des données expérimentales a été restreinte aux valeurs d'expression des microARNs (matures et précurseurs), soit 3391 identifiants (ensembles de sondes) parmi les 5683 identifiants spécifiques de l'humain. Dans le but d'éliminer les niveaux d'expression trop faibles et ainsi de limiter le bruit de fond, les données expérimentales ont été filtrées. Seuls ont été conservés pour l'analyse les gènes ayant au moins 5 de leurs valeurs d'expression supérieures au seuil fixé soit au moins 5/7 HV ou 5/8 RI ou 5/9 RII. Dans un premier temps, la gamme dynamique représentant le nombre d'identifiants en fonction du niveau d'expression a été réalisée, et un histogramme a permis d'estimer le seuil moyen pour dépasser le bruit de fond.

Plusieurs filtres ont été testés :

- $Log_2$(Expression) supérieure ou égale à 1 soit une Expression supérieure ou égale à 2,
- $Log_2$(Expression) supérieure ou égale à 1,5 soit une Expression supérieure ou égale à 3,
- $Log_2$(Expression) supérieure ou égale à 2 soit une Expression supérieure ou égale à 4,
- $Log_2$(Expression) supérieure ou égale à 2,5 soit une Expression supérieure ou égale à 6,
- $Log_2$(Expression) supérieure ou égale à 3 soit une Expression supérieure ou égale à 8.

Pour comparer les résultats obtenus au moyen de ces différents filtres, les analyses différentielles (individus RII versus individus sains) ont été réalisées. Ces analyses ont permis d'établir des listes de microARNs modulés de manière significative, c'est-à-dire respectant les critères de sélection énoncés ci-après :

- Un taux de faux positifs inférieur ou égal à 5% (FDR ≤ 0,05)
- |fold| ≥ 1,5

Le tableau 5 ci-après résume les résultats obtenus.

**Tableau 5 : Résultats de l'analyse différentielle RII vs HV après normalisation RMA et filtration des niveaux d'expression**

| Filtre | Nombre d'ensemble de sondes (IDs) | | Analyse différentielle RII vs HV | | |
|---|---|---|---|---|---|
| | Avant filtre sur l'expression | Après filtre sur l'expression | microARNs modulés* (pré + matures) | microARNs matures modulés* | microARNs matures modulés** |
| **Sans filtre $Log_2$ (expression)** | 3391 | 3391 (100%) | 39 (22 + / 17-) | 29 (12 + / 17-) | 160 |

(suite)

| Filtre | Nombre d'ensemble de sondes (IDs) | | Analyse différentielle RII vs HV | | |
|---|---|---|---|---|---|
| | Avant filtre sur l'expression | Après filtre sur l'expression | microARNs modulés* (pré + matures) | microARNs matures modulés* | microARNs matures modulés** |
| $Log_2$ (expression) $\geq$ 1 | 3391 | 2878 (85%) | 42 (23 + / 19-) | 33 (14 + / 19-) | 160 |
| $Log_2$ (expression) $\geq$ 1,5 | 3391 | 1355 (40%) | 81 (43 + / 38-) | 63 (28 + / 35-) | 160 |
| $Log_2$ (expression) $\geq$ 2 | 3391 | 937 (28%) | 102 (50 + / 52-) | 81 (32 + / 49-) | 160 |
| $Log_2$ (expression) $\geq$ 2,5 | 3391 | 757 (22%) | 98 (46 + / 52-) | 83 (33 + / 50-) | 160 |
| $Log_2$ (expression) $\geq$ 3 | 3391 | 648 (19%) | 100 (48 + / 52-) | 85 (35 + / 50-) | 160 |
| *microARNs humains avec \|Fold\| $\geq$ 1,5 et FDR $\leq$ 0,05 **microARNs humains avec \|Fold\| $\geq$ 1,5 et p-value $\leq$ 0,05 | | | | | |

Il est important de noter que les différences trouvées en jouant avec les divers filtres portent uniquement sur les valeurs du FDR. En effet, ni la p-value ni le fold modulation ne sont impactés. Pour illustrer l'importance du FDR, la liste de microARNs matures modulés de manière significative a été établie en se basant sur une p-value$\leq$0,05 et |Fold|$\geq$1,5. Par cette méthode, 160 microARNs matures sont modulés ; cela correspond à un taux de fausses découvertes de 50% si on se base sur la liste de 81 microARNs matures modulés (FDR$\leq$0,05 et |Fold|$\leq$1,5) avec le filtre $log_2$(Expression)$\geq$2. Seuls les microARNs matures ont été considérés dans la mesure où ce sont eux qui assurent la fonction biologique de régulation des ARNm. Par ailleurs, pour ces microARNs, le signal correspondant à leurs précurseurs respectifs était trop faible pour passer le filtre sur le niveau d'expression.

Les intersections entre les listes de microARNs matures modulés de manière significative ont été réalisées. Parmi les 81 microARNs matures trouvés modulés de manière significative avec le filtre $Log_2$(expression)$\geq$2 :

- On retrouve les 29 trouvés modulés de manière significative sans appliquer de filtre sur les niveaux d'expression ; il n'y a donc pas de perte d'information
- 70 microARNs sont en commun avec le filtre plus stringent $Log_2$(expression) $\geq$ 3 ; il y a donc un gain d'information par ajout de 41 microARNs à la liste initiale de 29 sans filtre.

Par ailleurs, si on s'intéresse aux microARNs qui divergent entre les listes établies avec les filtres $Log_2$(expression)$\geq$ 2 et $Log_2$(expression)$\geq$ 3, on s'aperçoit que :

- 8 microARNs sont inclus uniquement dans la liste avec le filtre $Log_2$(expression)$\geq$ 2; leurs niveaux d'expression ne passent pas le filtre $Log_2$(expression)$\geq$ 3 ce qui signifie que l'on perd de l'information,
- 15 microARNs sont inclus uniquement dans la liste avec le filtre $Log_2$(expression)$\geq$ 3; leurs FDR sont comprises entre 0,05 et 0,06 quand le filtre $Log_2$(expression)$\geq$ 2 a été appliqué.

En conclusion, dans cette étude, le filtre $Log_2$(expression)$\geq$ 2 a été retenu pour éliminer un maximum de bruit de fond sans toutefois perdre d'information avant de procéder aux analyses différentielles.

*d) Liste de microARNs modulés de manière significative dans RII vs HV*

Parmi les 81 mictroARNs modulés de manière significative avec FDR $\geq$ 0,05 et |fold|$\geq$ 1,5 : 32 sont surexprimés et 49 sous-exprimés chez RII.

**Tableau 6 : Liste de microARNs sous-exprimés (panneau gauche) et surexprimés (panneau droit) dans RII par rapport aux individus sains**

| probe set id | Fold | FDR | probe set id | Fold | FDR |
|---|---|---|---|---|---|
| hsa-miR-133a | -45,8 | 4,74E-02 | hsa-miR-3201 | 4,9 | 9,00E-04 |
| hsa-miR-133b | -39,0 | 3,08E-02 | hsa-miR-4423-3p | 4,4 | 9,00E-04 |
| hsa-miR-1 | -9,7 | 2,50E-02 | hsa-miR-3128 | 3,0 | 1,00E-03 |
| hsa-miR-299-5p | -5,1 | 3,09E-02 | hsa-miR-155 | 2,4 | 2,73E-02 |
| hsa-miR-486-3p | -4,7 | 4,24E-02 | hsa-miR-3163 | 2,4 | 1,02E-02 |
| hsa-miR-381 | -3,5 | 4,82E-02 | hsa-miR-146b-5p | 2,3 | 8,50E-03 |
| hsa-miR-4324 | -3,5 | 1,60E-02 | hsa-miR-606 | 2,2 | 3,61E-02 |
| hsa-miR-154 | -3,5 | 6,00E-04 | hsa-miR-150 | 2,1 | 3,56E-02 |
| hsa-miR-1247 | -3,4 | 8,90E-03 | hsa-miR-4776-5p | 2,1 | 9,00E-04 |
| hsa-miR-1287 | -3,3 | 1,74E-02 | hsa-miR-3124-5p | 1,9 | 2,76E-02 |
| hsa-miR-376c | -2,8 | 4,50E-02 | hsa-miR-4741 | 1,9 | 1,80E-02 |
| hsa-miR-195* | -2,8 | 1,28E-02 | hsa-miR-635 | 1,9 | 2,56E-02 |
| hsa-miR-4269 | -2,6 | 1,20E -03 | hsa-miR-4668-5p | 1,8 | 8,50E-03 |
| hsa-miR-1296 | -2,5 | 6,70E-03 | hsa-miR-4763-3p | 1,8 | 2,76E-02 |
| hsa-miR-34c-3p | -2,5 | 1,80E-03 | hsa-miR-4417 | 1,8 | 4,24E-02 |
| hsa-miR-204 | -2,5 | 2,13E-02 | hsa-miR-1911* | 1,8 | 4,55E-02 |
| hsa-miR-504 | -2,5 | 2,80E-02 | hsa-miR-4734 | 1,7 | 2,76E-02 |
| hsa-miR-30c-1* | -2,5 | 8,30E-03 | hsa-miR-4530 | 1,7 | 4,20E-02 |
| hsa-miR-615-3p | -2,4 | 2,70E-03 | hsa-miR-4260 | 1,7 | 3,86E-02 |
| hsa-miR-505 | -2,3 | 4,24E-02 | hsa-miR-3185 | 1,7 | 4,24E-02 |
| hsa-miR-508-5p | -2,3 | 5,90E-03 | hsa-miR-4707-5p | 1,7 | 4,24E-02 |
| hsa-miR-409-5p | -2,3 | 4,36E-02 | hsa-miR-4745-5p | 1,7 | 3,50E-02 |
| hsa-miR-433 | -2,2 | 3,50E-02 | hsa-miR-4659a-3p | 1,7 | 1,28E-02 |
| hsa-miR-375 | -2,2 | 1,88E-02 | hsa-miR-1469 | 1,7 | 1,45E-02 |
| hsa-miR-935 | -2,2 | 1,72E-02 | hsa-miR-4695-5p | 1,7 | 1,88E-02 |
| hsa-miR-128 | -2,1 | 2,73E-02 | hsa-miR-4674 | 1,6 | 3,50E-02 |
| hsa-miR-331-5p | -2,1 | 4,44E-02 | hsa-miR-4687-3p | 1,6 | 6,60E-03 |
| hsa-miR-584 | -2,0 | 4,02E-02 | hsa-miR-4739 | 1,6 | 3,29E-02 |
| hsa-miR-30a* | -1,9 | 1,00E-03 | hsa-miR-1915 | 1,6 | 3,50E-02 |
| hsa-miR-370 | -1,9 | 6,50E-03 | hsa-miR-216b | 1,6 | 4,15E-02 |
| hsa-miR-148a* | -1,9 | 3,68E-02 | hsa-miR-548a-3p | 1,5 | 4,00E-02 |
| hsa-miR-489 | -1,9 | 4,24E-02 | | | |
| hsa-miR-30e* | -1,8 | 4,24E-02 | | | |
| hsa-miR-337-5p | -1,8 | 3,01E-02 | | | |
| hsa-miR-1181 | -1,8 | 4,55E-02 | | | |
| hsa-miR-1237 | -1,8 | 3,61E-02 | | | |
| hsa-miR-485-5p | -1,7 | 2,76E-02 | | | |
| hsa-miR-23c | -1,7 | 4,20E-02 | | | |
| hsa-miR-148b | -1,7 | 1,66E-02 | | | |
| hsa-miR-378g | -1,7 | 2,26E-02 | | | |
| hsa-miR-1238 | -1,7 | 4,55E-02 | | | |
| hsa-miR-181a-2* | -1,6 | 3,50E-02 | | | |
| hsa-miR-187 | -1,6 | 4,20E-02 | | | |
| hsa-miR-378d | -1,6 | 1,51E-02 | | | |
| hsa-miR-181d | -1,6 | 2,69E-02 | | | |
| hsa-miR-98 | -1,5 | 4,24E-02 | | | |
| hsa-miR-4685-3p | -1,5 | 4,24E-02 | | | |
| hsa-miR-23b* | -1,5 | 4,24E-02 | | | |

e) Clustering hiérarchique

Dans le but de vérifier si les 81 microARNs matures exprimés différentiellement dans RII d'après la technologie Affymetrix permettent de bien différencier les individus malades des individus sains, un clustering hiérarchique a été réalisé dans Array studio (résultats non montrés).

Deux groupes caractéristiques sont mis en évidence :

- Un groupe composé de 6 sujets sains
- Un groupe composé de 9 sujets atteints de Rosacée de sous-type II et d'un sujet sain.

Ce sujet sain montre un schéma de profil d'expression atypique, ne s'apparentant, ni à celui des RII ni à celui des HV. Par ailleurs, lors d'une étude précédente, cet individu avait également été classé dans un groupe de sujets malades lors de la réalisation du clustering pour les ARNm. En conclusion, malgré un individu discordant, les profils d'expression des microARNs permettent bien de différencier les individus malades des individus sains.

microARN(s) biomarqueur(s)

Afin de mettre en évidence de potentiels microARNs biomarqueurs de la Rosacée PPR, l'intersection des listes de microARNs significativement modulés dans 3 études de caractérisation du miRNome dans des pathologies de la peau a été faite. Ces études concernent :

- le présent projet : caractérisation du miRNome dans la Rosacée (RII versus HV).
- une autre étude: caractérisation du miRNome dans de la peau lésionnelle acnéique (étude réalisée dans les mêmes conditions expérimentales que l'étude sur la Rosacée) versus de la peau non lésionnelle.
- une étude externe : caractérisation du miRNome dans le psoriasis (Zibert et al. J Dermatol Sci. 2010 58(3):177-85) réalisées avec des technologies différentes de celles utilisées (panel plus restreint de microARNs étudiés).

Les résultats de ces intersections ont montré une proportion importante de microARNs s'exprimant de façon spécifique pour chacune de ces pathologies.

Ainsi, 76 microARNs s'exprimeraient de façon spécifique dans la Rosacée de type II. Parmi ces 76 microARNs, 27 sont surexprimés dans RII (par rapport aux HV). Un biomarqueur doit préférablement être fortement exprimé dans la pathologie et faiblement voire non exprimé chez les individus sains. En se basant à la fois sur le fold modulation (le plus élevé possible) et l'expression dans la peau saine (la plus faible possible), une liste de 7 biomarqueurs potentiels de la Rosacée de type II a été établie. Cette liste comprend les microARNs hsa-miR-606, hsa-miR-635, hsa-miR-3128, hsa-miR-3163, hsa-miR-3201, hsa-miR-4423-3p et hsa-miR-4776-5p (encadrés ci-dessous).

**Tableau 7: Liste de 27 microARNs surexprimés dans RII (non modulés dans les études acné et psoriasis)**

| miRBase (mature) | RII vs HV Fold Change | Expression moyenne HV (étude Rosacée) | Expression moyenne RII | RII vs RI Fold Change | Expression moyenne RI | acne vs HV Fold Change | Expression moyenne HV (étude acné) |
|---|---|---|---|---|---|---|---|
| hsa-miR-3201 | 4,9 | 51 | 248 | 2,4 | 102* | -1,1 | 46 |
| hsa-miR-4423-3p | 4,4 | 9 | 38 | 1,9 | 20* | -1,3 | 10 |
| hsa-miR-3128 | 3,0 | 34 | 102 | 2,2 | 47 | -1,1 | 18 |
| hsa-miR-3163 | 2,4 | 4 | 10 | 1,9 | 6 | -1,2 | 5 |
| hsa-miR-606 | 2,2 | 3 | 7 | 1,6 | 5 | ne passe pas filtre d'expression | |
| hsa-miR-4776-5p | 2,1 | 8 | 17 | 1,5 | 11 | -1,2 | 5 |

(suite)

| miRBase (mature) | RII vs HV Fold Change | Expression moyenne HV (étude Rosacée) | Expression moyenne RII | RII vs RI Fold Change | Expression moyenne RI | acne vs HV Fold Change | Expression moyenne HV (étude acné) |
|---|---|---|---|---|---|---|---|
| hsa-miR-635 | 1,9 | 8 | 14 | 1,3 | 11 | 1,1 | 5 |
| hsa-miR-3124-5p | 1,9 | 46 | 88 | 1,3 | 69 | -1,2 | 26 |
| hsa-miR-4741 | 1,9 | 288 | 537 | 1,2 | 439* | -1,4 | 397 |
| hsa-miR-4668-5p | 1,8 | 1388 | 2476 | 1,2 | 2027 | -1,3 | 1489 |
| hsa-miR-4763-3p | 1,8 | 1235 | 2182 | 1,2 | 1889* | -1,3 | 1756 |
| hsa-miR-1911* | 1,8 | 3 | 5 | 1,1 | 4* | ne passe pas filtre d'expression | |
| hsa-miR-4734 | 1,7 | 855 | 1495 | 1,1 | 26* | -1,4 | 1196 |
| hsa-miR-4530 | 1,7 | 786 | 1371 | 1,5 | 906 | -1,3 | 1091 |
| hsa-miR-4260 | 1,7 | 3 | 6 | 1,4 | 4 | ne passe pas filtre d'expression | |
| hsa-miR-3185 | 1,7 | 479 | 816 | 1,1 | 743* | -1,3 | 628 |
| hsa-miR-4707-5p | 1,7 | 903 | 1535 | 1,1 | 1353* | -1,3 | 1200 |
| hsa-miR-4745-5p | 1,7 | 1374 | 2321 | 1,2 | 1956 | -1,3 | 1518 |
| hsa-miR-4659a-3p | 1,7 | 2 | 4 | 1,5 | 3 | ne passe pas filtre d'expression | |
| hsa-miR-1469 | 1,7 | 2171 | 3606 | 1,2 | 3000 | -1,2 | 2692 |
| hsa-miR-4695-5p | 1,7 | 325 | 537 | 1,3 | 416 | -1,2 | 299 |
| hsa-miR-4674 | 1,6 | 388 | 640 | 1,2 | 549 | -1,4 | 470 |
| hsa-miR-4687-3p | 1,6 | 2168 | 3569 | 1,3 | 2748 | -1,2 | 2724 |
| hsa-miR-4739 | 1,6 | 753 | 1221 | 1,2 | 1005 | -1,1 | 929 |
| hsa-miR-1915 | 1,6 | 3941 | 6301 | 1,2 | 5260 | -1,3 | 5594 |
| hsa-miR-216b | 1,6 | 3 | 5 | 1,3 | 4 | ne passe pas filtre d'expression | |
| hsa-miR-548a-3p | 1,5 | 23 | 35 | 1,1 | 32 | -1,1 | 19 |

*|Fold change Expression moyenne| ≥ 1,5 in RI vs HV

***Matériel et méthodes***

Extraction des ARN totaux (ARNm et microARNs)

L'extraction des ARN totaux a été réalisée auparavant afin de réaliser l'étude du transcriptome. Cette extraction a été réalisée avec le kit miRNeasy Mini de QIAGEN qui permet d'extraire les grands ARNs (ARNm) ainsi que les petits ARNs incluant les microARNs. Ce kit comporte trois étapes successives : la lyse des tissus pour libérer les ARN contenus dans les cellules, l'isolation des ARNs sur membrane puis leur élution. Les résultats de l'étude transcriptomique précédemment établis ont été utilisés pour l'interprétation biologique des données sur les microARNs obtenues.

Etude à large échelle des microARNs

*Etude de l'expression des gènes de microARNs : puce Affymetrix miRNA*

La puce miRNA 3.0 est composée des fragments d'acides nucléiques, appelés sondes, liées au support physique et dont l'ensemble des séquences correspond aux microARNs de 153 organismes dont plus de 1700 formes matures de microARNs humains. Chaque sonde (fragment d'ADN simple brin) est complémentaire d'un microARN donné. Pour chaque microARN, plusieurs sondes, variant parfois de quelques paires de bases, sont présentes sur la puce. C'est ce qu'on appelle un ensemble de sondes. La puce miRNA 3.0 contient près de 20 000 ensembles de sondes dont plus de 5 600 spécifiques aux petits ARNs humains dont les microARNs (1 733 matures et 1 658 précurseurs). Le design de cette puce a été réalisé à partir de miRBase version 17. Un ensemble de sondes dédié à un microARN mature est composé de 9 sondes identiques, complémentaires à la séquence de l'ARN mature cible.

L'expérience est faite à partir de 300 ng d'ARN totaux. La première étape consiste à ajouter une queue poly(A) aux microARNs. Cette queue poly(A), initialement présente sur les ARNm et dorénavant également sur les microARNs, va permettre la fixation de la molécule 3DNA® étiquetée avec 15 biotines. On aboutit alors à la formation d'ARN biotinylés. La deuxième étape est l'hybridation des ARN sur leurs sondes respectives ; les sondes présentes dans les différents compartiments de la puce étant spécifiques des microARNs, les ARNm biotinylés se retrouvent excluent tandis que les microARNs biotinylés vont s'hybrider aux sondes. Un lavage permet d'éliminer les molécules qui ne se sont pas hybridées sur la puce. Dans une troisième étape, la streptavidine couplée à une molécule fluorescente, la phycoerythrine est ajoutée. Grâce à son affinité pour la biotine, la streptavidine va permettre de mettre en évidence les microARNs biotinylés. Afin d'optimiser ce marquage, une amplification du marquage est réalisée : pour cela, un anticorps de chèvre anti-streptavidine couplé à une biotine est introduit. Ce dernier va se fixer au complexe précédent microARN biotinylé-Streptavidine/phycoerythrine. L'ajout, une deuxième fois, de streptavidine/phycoerythrine, va avoir pour effet d'amplifier le marquage fluorescent afin d'améliorer la détection. Les étapes de marquage et lavages successifs sont automatisées grâce à une plateforme Affymetrix dédiée. Le signal fluorescent est ensuite traité au moyen du logiciel Expression console, fourni par Affymetrix. Plus un microARN est fixé en grande quantité sur sa sonde complémentaire, plus le signal lumineux sera intense. Ainsi, pour chaque puce, c'est en comparant l'intensité lumineuse correspondant à chaque sonde d'un échantillon traité versus un échantillon témoin que l'on peut analyser l'effet de ce traitement sur l'expression des gènes.

*Biostatistiques*

Sur un plan technique, les études de transcriptomique sont à la fois fiables et associées à des coûts abordables. Toutefois, l'analyse des quantités importantes de données générées demeure un point crucial. Dans ce domaine, le recours aux outils bioinformatiques et aux biostatistiques est indispensable. Il n'y a pas toujours de consensus concernant le choix des algorithmes mathématiques utilisés pour traiter les données. Pour les études du miRNome notamment, à large échelle ou à plus petite échelle par qPCR, plusieurs méthodes de normalisation des données brutes ont été proposées. Concernant les études à large échelle de l'expression des microARNs, la méthode la plus utilisée actuellement est la normalisation RMA. Avant normalisation, les données d'expression sont transformées en $\log_2$ afin d'aboutir à une gamme linéaire et de réduire l'étendue des données.

Logiciel Array studio : Array studio est un package logiciel conçu pour les biologistes et bioinformaticiens afin d'analyser statistiquement des données de séquençage nouvelle génération, de SNPs ou encore de microarray. Il inclut notamment le support pour toutes les plateformes microarray dont Agilent, Illumina et Affymetrix, cette dernière étant utilisée dans le cadre de notre étude. Il permet dans un premier temps de normaliser les données brutes selon diverses méthodes, puis dans un second temps de réaliser des analyses différentielles inter-groupes pour comparer les profils d'expression des microARNs.

Normalisation RMA (Robust Multiarray/Multichip Average) : Les études à large échelle rendent désormais possible l'analyse des profils d'expression de nombreux gènes en une seule expérience ; par exemple pour caractériser le miRNome d'un individu. Ces expériences, répétées pour chaque échantillon (individu) testé, génèrent beaucoup de données mais ces données peuvent être biaisées par des variations d'origine technologique. Le but de la normalisation est d'enlever le bruit lié à la technologie afin de prendre en compte au plus précisément les variations d'origine biologique. La normalisation RMA comporte trois étapes successives : la correction du bruit de fond; la normalisation par quantiles : elle s'effectue sur l'ensemble des puces de l'étude considérée et tend à homogénéiser les distributions des niveaux d'expression entre les puces afin de les analyser conjointement, ou encore les comparer; et le « median polish » : étape au cours de laquelle les intensités de fluorescence des cibles se fixant sur un même ensemble de sondes sont combinées afin d'estimer une valeur d'intensité unique, par puce, pour chaque ensemble de cibles (ou gène).

Analyses différentielles

Une fois les niveaux d'expression normalisés, des analyses différentielles inter-groupes permettent de comparer :

- les individus issus du sous-groupe de rosacée I au sous-groupe d'individus sains,
- les individus issus du sous-groupe de rosacée II au sous-groupe d'individus sains,
- les individus issus du sous-groupe de rosacée II à ceux issus du sous-groupe de rosacée I.

Ainsi, la caractérisation du miRNome dans une condition pathologique se fait par comparaison aux profils des microARNs obtenus dans une condition saine. Le principe de cette comparaison repose sur le calcul du « fold modulation » (modulation du niveau d'expression d'un gène). Pour connaître de degré de confiance accordé à ce fold, une p-value lui est associée, calculée grâce à un test d'hypothèses appelé test de Student.

*Calcul du « fold modulation »*

a) Expression relative des données à large échelle (Affymetrix)

Comme les données d'expression normalisées ont été transformées en log2, l'expression relative se calcule de la manière suivante :

$$\text{Expression relative} = 2^{\Delta}$$

avec

$$\Delta = \log_2(\text{Expression moyenne }_{\text{gène i condition B}}) - \log_2(\text{Expression moyenne }_{\text{gène i condition A/référence}})$$

b) Facteur de modulation ou « Fold »

Le « fold modulation » se traduit par la modulation de la moyenne d'expression du gène *i* dans la condition A (de référence) par rapport à la condition B; si le fold est positif alors le gène *i* est surexprimé dans la condition A par rapport à la condition B contrôle. A l'inverse, si le fold est négatif alors le gène *i* est sous-exprimé dans la condition A par rapport à la condition B contrôle. Dans le cas où la valeur d'expression relative calculée est supérieure à 1, elle équivaut à un facteur de modulation (« Fold ») positif. En revanche, dans le cas où l'expression relative calculée est comprise entre 0 et 1, signifiant que le gène est plus exprimé dans la condition de référence (A) que dans la condition testée (B), on réalise alors l'opération -1/fold pour obtenir des Folds négatifs, plus facilement interprétables. Un seuil sur le fold peut être fixé par le biologiste pour sélectionner spécifiquement ceux ayant un intérêt d'un point de vue biologique ; dans cette étude, il est fixé à +/-1,5.

*Principe d'un test d'hypothèses*

En statistique, un test d'hypothèses consiste à évaluer une hypothèse statistique en fonction de jeu de données (échantillon). Par exemple, si on pose la question : Est-ce que le gène *i* est différentiellement exprimé dans la condition A par rapport à la condition B ? La condition A peut correspondre à des individus malades et la condition B des individus sains contrôles.

Répondre par oui ou non à la question revient à trancher entre deux hypothèses :

- Hypothèse nulle ou H0 : le gène n'est pas différentiellement exprimé,
- Hypothèse alternative ou H1 : le gène est différentiellement exprimé.

Deux types d'erreurs peuvent se poser :

- Erreur de type I : rejeter H0 alors que H0 est vraie, revient à considérer que le gène est différentiellement exprimé alors qu'il ne l'est pas; il s'agit alors d'un faux positif.
- Erreur de type II : ne pas rejeter H0 alors que H0 est fausse, revient à considérer que le gène n'est pas différentiellement exprimé alors qu'il l'est en réalité ; il s'agit dans ce cas d'un faux négatif.

Le risque maximum toléré (noté a) est appelée seuil de significativité ; il est généralement fixée à 0,05. Dans notre exemple, cela revient en moyenne à accepter le risque de se tromper 5 fois sur 100, et donc d'accepter en moyenne 5% de faux positifs dans notre analyse.

La statistique du test est calculée à partir des données. Sa valeur permet d'estimer le pourcentage de chance (p-value) nécessaire pour obtenir ces données si H0 est vraie. En fonction du seuil de significativité préalablement fixé et de cette p-value, la décision de rejeter ou non H0 peut être prise.

*Test de Student ou t-test*

Le test permet d'évaluer si les moyennes d'expression du gène *i* dans chaque condition A et B sont statistiquement différentes l'une de l'autre et ainsi de comparer une condition par rapport à une autre. La p-value du t-test permet ensuite de conclure si la différence entre les moyennes n'est pas due au hasard :

- Si p-value $\leq$ 0,05 alors on rejette H0; les moyennes des deux conditions A et B sont dîtes significativement différentes, le gène *i* est donc modulé de manière significative dans la condition A par rapport à la condition B.
- Si p-value > 0.05 alors on ne rejette pas H0 ; les moyennes des deux conditions A et B sont dîtes non-significativement différentes, le gène *i* n'est donc pas modulé de manière significative dans la condition A par rapport à la condition B.

*Contrôle du taux de fausses découvertes ou FDR*

Les puces d'expression des analyses à large échelle permettent de mesurer simultanément l'activité transcriptionnelle de plusieurs milliers de gènes pour un échantillon biologique donné. Pour les analyser de manière simultanée, on multiplie les tests d'hypothèses. Toutefois, lorsque l'on multiplie les tests, la probabilité de détecter un gène significativement modulé, alors qu'il ne l'est pas, augmente. Pour contrôler le taux de fausses découvertes (gènes considérés modulés alors qu'ils ne le sont pas), on contrôle le taux de fausses découvertes grâce à une méthode mise au point par Benjamini et Hochberg, 1995, Journal of the Royal Statistical Society. Series B (Methodological) vol. 57, No. 1 (1995), pp. 289-300, le FDR (False Discovery Rate) qui corrige les p-values associées aux t-tests afin de conserver le même taux de fausses découvertes quel que soit le nombre de gènes. En conclusion, après une analyse différentielle, le gène *i* est considéré significativement modulé s'il respecte deux conditions :

- |fold| $\geq$ 1,5
- FDR < 0,05

## Revendications

1. Méthode pour diagnostiquer une rosacée de type II chez un sujet, comprenant la détermination dans un échantillon de derme et/ou épiderme dudit sujet de l'expression d'au moins la combinaison des sept microARNs suivants : hsa-miR-3201, hsa-miR-4423-3p, hsa-miR-3128, hsa-miR-3163, hsa-miR-606, hsa-miR-4776-5p, et hsa-miR-635, et dans laquelle une expression desdits microARNs dudit échantillon supérieure à celle d'échantillons contrôles indique que le sujet souffre de ladite rosacée.

2. Méthode selon la revendication 1, comprenant la détermination supplémentaire de l'expression d'un ou plusieurs microARNs choisis parmi

hsa-miR-133a      hsa-miR-3201

(suite)

| | |
|---|---|
| hsa-miR-133b | hsa-miR-4423-3p |
| hsa-miR-1 | hsa-miR-3128 |
| hsa-miR-299-5p | hsa-miR-155 |
| hsa-miR-486-3p | hsa-miR-3163 |
| hsa-miR-381 | hsa-miR-146b-5p |
| hsa-miR-4324 | hsa-miR-606 |
| hsa-miR-154 | hsa-miR-150 |
| hsa-miR-1247 | hsa-miR-4776-5p |
| hsa-miR-1287 | hsa-miR-3124-5p |
| hsa-miR-376c | hsa-miR-4741 |
| hsa-miR-195* | hsa-miR-635 |
| hsa-miR-4269 | hsa-miR-4668-5p |
| hsa-miR-1296 | hsa-miR-4763-3p |
| hsa-miR-34c-3p | hsa-miR-4417 |
| hsa-miR-204 | hsa-miR-1911* |
| hsa-miR-504 | hsa-miR-4734 |
| hsa-miR-30c-1* | hsa-miR-4530 |
| hsa-miR-615-3p | hsa-miR-4260 |
| hsa-miR-505 | hsa-miR-3185 |
| hsa-miR-508-5p | hsa-miR-4707-5p |
| hsa-miR-409-5p | hsa-miR-4745-5p |
| hsa-miR-433 | hsa-miR-4659a-3p |
| hsa-miR-375 | hsa-miR-1469 |
| hsa-miR-935 | hsa-miR-4695-5p |
| hsa-miR-128 | hsa-miR-4674 |
| hsa-miR-331-5p | hsa-miR-4687-3p |
| hsa-miR-584 | hsa-miR-4739 |
| hsa-miR-30a* | hsa-miR-1915 |
| hsa-miR-370 | hsa-miR-216b |
| hsa-miR-148a* | hsa-miR-548a-3p |
| hsa-miR-489 | |
| hsa-miR-30e* | |
| hsa-miR-337-5p | |
| hsa-miR-1181 | |
| hsa-miR-1237 | |
| hsa-miR-485-5p | |
| hsa-miR-23c | |
| hsa-miR-148b | |
| hsa-miR-378g | |
| hsa-miR-1238 | |
| hsa-miR-181a-2* | |
| hsa-miR-187 | |
| hsa-miR-378d | |
| hsa-miR-181d | |
| hsa-miR-98 | |
| hsa-miR-4685-3p | |
| hsa-miR-23b* | |

3. Méthode selon la revendication 1 ou 2, comprenant la détermination supplémentaire de l'expression d'un ou plusieurs microARNs choisis dans un des groupes

a) hsa-miR-133a, hsa-miR-133b, hsa-miR-1, hsa-miR-299-5p, hsa-miR-486-3p, hsa-miR-381, hsa-miR-4324, hsa-miR-154, hsa-miR-1247, hsa-miR-1287, hsa-miR-376c, hsa-miR-195*, hsa-miR-4269, hsa-miR-1296, hsa-miR-34c-3p, hsa-miR-204, hsa-miR-504, hsa-miR-30c-1*, hsa-miR-615-3p, hsa-miR-505, hsa-miR-508-5p, hsa-miR-409-5p, hsa-miR-433, hsa-miR-375, hsa-miR-935, hsa-miR-128, hsa-miR-331-5p, hsa-miR-584, hsa-miR-30a*, hsa-miR-370, hsa-miR-148a*, hsa-miR-489, hsa-miR-30e*, hsa-miR-337-5p, hsa-miR-1181, hsa-miR-1237, hsa-miR-485-5p, hsa-miR-23c, hsa-miR-148b, hsa-miR-378g, hsa-miR-1238, hsa-miR-181a-2*, hsa-miR-187, hsa-miR-378d, hsa-miR-181d, hsa-miR-98, hsa-miR-4685-3p, hsa-miR-23b*, hsa-miR-3201, hsa-miR-4423-3p, hsa-miR-3128, hsa-miR-155, hsa-miR-3163, hsa-miR-146b-5p, hsa-miR-606, hsa-miR-150, hsa-miR-4776-5p, hsa-miR-3124-5p, hsa-miR-4741, hsa-miR-635, hsa-miR-4668-5p, hsa-miR-4763-3p, hsa-miR-4417, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

b) hsa-miR-223, hsa-miR-142-3p, hsa-miR-146b, hsa-miR-21, hsa-miR-331, hsa-miR-598, hsa-miR-24, hsa-miR-23a, hsa-miR-99a, hsa-miR-152, hsa-miR-423-5p, hsa-miR-34c, hsa-miR-27a, hsa-miR-30c, hsa-miR-125a-5p, mmu-miR-491, hsa-miR-127, hsa-miR-375, hsa-miR-101, hsa-miR-296, mmu-miR-379, hsa-miR-574-3p, hsa-miR-487b, mmu-miR-499, hsa-miR-95, hsa-miR-885-5p, hsa-miR-486-3p, hsa-miR-1, hsa-miR-133b, hsa-miR-133a

c) hsa-miR-133a, hsa-miR-133b, hsa-miR-1, hsa-miR-299-5p, hsa-miR-486-3p, hsa-miR-381, hsa-miR-4324, hsa-miR-154, hsa-miR-1247, hsa-miR-885-5p, hsa-miR-1287, hsa-miR-95, hsa-miR-376c, hsa-miR-195*, hsa-miR-4269, mmu-miR-499, hsa-miR-1296, hsa-miR-34c-3p, hsa-miR-204, hsa-miR-504, hsa-miR-30c-1*, hsa-miR-487b, hsa-miR-615-3p, hsa-miR-505, hsa-miR-508-5p, hsa-miR-409-5p, hsa-miR-433, hsa-miR-375, hsa-miR-935, hsa-miR-128, hsa-miR-331-5p, hsa-miR-584, hsa-miR-30a*, hsa-miR-370, hsa-miR-148a*, hsa-miR-489, hsa-miR-30e*, hsa-miR-337-5p, hsa-miR-1181, hsa-miR-1237, hsa-miR-485-5p, hsa-miR-23c, hsa-miR-491-5p, hsa-miR-148b, hsa-miR-378g, hsa-miR-125a-5p, hsa-miR-1238, hsa-miR-181a-2*, hsa-miR-187, hsa-miR-30c, hsa-miR-27a, hsa-miR-99a, hsa-miR-378d, hsa-miR-181d, hsa-miR-98, hsa-miR-4685-3p, hsa-miR-598, hsa-miR-23b*, hsa-miR-3201, hsa-miR-4423-3p, hsa-miR-223, hsa-miR-3128, hsa-miR-155, hsa-miR-3163, hsa-miR-146b-5p, hsa-miR-606, hsa-miR-150, hsa-miR-4776-5p, hsa-miR-142-3p, hsa-miR-21, hsa-miR-3124-5p, hsa-miR-4741, hsa-miR-635, hsa-miR-4668-5p, hsa-miR-4763-3p, hsa-miR-4417, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

d) hsa-miR-3201, hsa-miR-4423-3p, hsa-miR-3128, hsa-miR-3163, hsa-miR-606, hsa-miR-4776-5p, hsa-miR-635, hsa-miR-3124-5p, hsa-miR-4741, hsa-miR-4668-5p, hsa-miR-4763-3p, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p.

**4.** Méthode selon l'une des revendications 1 à 3, comprenant la détermination supplémentaire de l'expression d'un ou plusieurs microARNs choisis parmi hsa-miR-133a, hsa-miR-133b, hsa-miR-1, hsa-miR-486-3p, hsa-miR-34c-3p, hsa-miR-375, et hsa-miR-146b-5p.

**5.** Méthode selon la revendication 1, comprenant la détermination supplémentaire de l'expression d'un ou plusieurs microARNs choisis dans un des groupes consistant en

1) hsa-miR-4758-5p, hsa-miR-4665-5p, hsa-miR-149*, hsa-miR-548ac, hsa-miR-4507, hsa-miR-548a-3p, hsa-miR-4289, hsa-miR-4727-3p, hsa-miR-4468, hsa-miR-4463, hsa-miR-2861, hsa-miR-4773, hsa-miR-1825, hsa-miR-4651, hsa-miR-216b, hsa-miR-4689, hsa-miR-3152-3p, hsa-miR-4270, hsa-miR-1915, hsa-miR-4739, hsa-miR-4772-5p, hsa-miR-4687-3p, hsa-miR-4674, hsa-miR-4695-5p, hsa-miR-4439, hsa-miR-129-3p, hsa-miR-1469, hsa-miR-4659a-3p, hsa-miR-4799-3p, hsa-miR-4657, hsa-miR-4655-5p, hsa-miR-4745-5p, hsa-miR-4662b, hsa-miR-4707-5p, hsa-miR-3185, hsa-miR-4260, hsa-miR-4530, hsa-miR-4734, hsa-miR-1911*, hsa-miR-4417, hsa-miR-4763-3p, hsa-miR-4668-5p, hsa-miR-1281, hsa-miR-635, hsa-miR-4741, hsa-miR-3124-5p, hsa-miR-146b-3p, hsa-miR-4529-3p, hsa-miR-4776-5p, hsa-miR-150, hsa-miR-3927, hsa-miR-606, hsa-miR-146b-5p, hsa-miR-3163, hsa-miR-155, hsa-miR-371b-5p, hsa-miR-3128, hsa-miR-4423-3p, hsa-miR-335, hsa-miR-3201, hsa-miR-184

2) hsa-miR-1269b, hsa-miR-4741, hsa-miR-4763-3p, hsa-miR-4704-5p, hsa-miR-1911*, hsa-miR-3621, hsa-miR-3612, hsa-miR-4436b-5p, hsa-miR-4734, hsa-miR-3185, hsa-miR-3910, hsa-miR-4727-3p, hsa-miR-155, hsa-miR-155*, hsa-miR-4529-3p, hsa-miR-3927, hsa-miR-3201, hsa-miR-4423-3p, hsa-miR-124, hsa-miR-184;

3) hsa-miR-4776-5p, hsa-miR-601, hsa-miR-4530, hsa-miR-4773, hsa-miR-4717-3p, hsa-miR-4657, hsa-miR-4289, hsa-miR-4417, hsa-miR-4445*, hsa-miR-150, hsa-miR-146b-5p, hsa-miR-3175, hsa-miR-4646-5p, hsa-miR-3163, hsa-miR-4423-3p, hsa-miR-3128, hsa-miR-1244, hsa-miR-21*, hsa-miR-3201, hsa-miR-335

4) hsa-miR-223, hsa-miR-142-3p, hsa-miR-146b, hsa-miR-21, hsa-miR-155

a) hsa-miR-3201, hsa-miR-4423-3p, hsa-miR-3128, hsa-miR-155, hsa-miR-3163, hsa-miR-146b-5p, hsa-miR-606, hsa-miR-150, hsa-miR-4776-5p, hsa-miR-3124-5p, hsa-miR-4741, hsa-miR-635, hsa-miR-4668-5p, hsa-miR-4763-3p, hsa-miR-4417, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

b) hsa-miR-223, hsa-miR-142-3p, hsa-miR-146b, hsa-miR-21

c) hsa-miR-3201, hsa-miR-4423-3p, hsa-miR-223, hsa-miR-3128, hsa-miR-155, hsa-miR-3163, hsa-miR-146b-5p, hsa-miR-606, hsa-miR-150, hsa-miR-4776-5p, hsa-miR-142-3p, hsa-miR-21, hsa-miR-3124-5p, hsa-miR-4741, hsa-miR-635, hsa-miR-4668-5p, hsa-miR-4763-3p, hsa-miR-4417, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

d) hsa-miR-3201, hsa-miR-4423-3p, hsa-miR-3128, hsa-miR-3163, hsa-miR-606, hsa-miR-4776-5p, hsa-miR-635, hsa-miR-3124-5p, hsa-miR-4741, hsa-miR-4668-5p, hsa-miR-4763-3p, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

e) hsa-miR-146b-5p.

6. Méthode selon la revendication 1, comprenant la détermination supplémentaire dans un échantillon dudit sujet de l'expression d'un ou plusieurs microARNs choisis dans le groupe consistant en

1) hsa-miR-133a, hsa-miR-206, hsa-miR-133b, hsa-miR-1, hsa-miR-299-5p, hsa-miR-486-3p, hsa-miR-381, hsa-miR-4324, hsa-miR-154, hsa-miR-1247, hsa-miR-1287, hsa-miR-376c, hsa-miR-195*, hsa-miR-411, hsa-miR-4269, hsa-miR-328, hsa-miR-1296, hsa-miR-34c-3p, hsa-miR-204, hsa-miR-504, hsa-miR-30c-1*, hsa-miR-615-3p, hsa-miR-654-3p, hsa-miR-505, hsa-miR-508-5p, hsa-miR-409-5p, hsa-miR-338-5p, hsa-miR-433, hsa-miR-375, hsa-miR-935, hsa-let-7d*, hsa-miR-99a*, hsa-miR-128, hsa-miR-1290, hsa-miR-331-5p, hsa-miR-4730, hsa-miR-29c*, hsa-miR-455-5p, hsa-miR-377*, hsa-miR-378e, hsa-miR-584, hsa-miR-96, hsa-miR-30a*, hsa-miR-143*, hsa-miR-370, hsa-miR-148a*, hsa-miR-489, hsa-miR-506, hsa-let-7e*, hsa-miR-30e*, hsa-miR-4787-3p, hsa-let-7b*, hsa-miR-181c*, hsa-miR-513a-5p, hsa-miR-92a-1*, hsa-miR-337-5p, hsa-miR-1181, hsa-miR-1237, hsa-miR-3620, hsa-miR-485-5p, hsa-miR-23c, hsa-miR-18a*, hsa-miR-10a, hsa-miR-139-3p, hsa-miR-493, hsa-miR-148b, hsa-miR-550a, hsa-miR-3147, hsa-miR-378g, hsa-miR-675*, hsa-miR-1238, hsa-miR-542-5p, hsa-miR-30c-2*, hsa-miR-125a-3p, hsa-miR-181a-2*, hsa-miR-1292, hsa-miR-187, hsa-miR-378*, hsa-miR-493*, hsa-miR-495, hsa-miR-557, hsa-miR-3909, hsa-miR-378d, hsa-miR-491-5p, hsa-miR-181d, hsa-miR-671-3p, hsa-miR-513c, hsa-miR-487b, hsa-miR-378b, hsa-miR-885-5p, hsa-miR-98, hsa-miR-29b-2*, hsa-miR-4685-3p, hsa-miR-3605-3p, hsa-miR-24-1*, hsa-miR-4649-3p, hsa-miR-3180-5p, hsa-miR-149, hsa-miR-23b*

2) hsa-miR-206, hsa-miR-133b, hsa-miR-133a, hsa-miR-1, hsa-miR-486-3p, hsa-miR-299-5p, hsa-miR-1247, hsa-miR-381, hsa-miR-154, hsa-miR-433, hsa-miR-1244, hsa-miR-4485, hsa-miR-378e, hsa-miR-505, hsa-miR-376c, hsa-miR-1296, hsa-miR-29b-1*, hsa-miR-409-5p, hsa-miR-29c*, hsa-miR-34c-3p, hsa-miR-4646-5p, hsa-miR-99a*, hsa-miR-378g, hsa-miR-504, hsa-miR-584, hsa-miR-128, hsa-miR-30c-1*, hsa-miR-665, hsa-miR-4269, hsa-miR-493*, hsa-miR-328, hsa-miR-550a, hsa-miR-375, hsa-miR-493, hsa-miR-378d, hsa-miR-148a*, hsa-miR-615-3p, hsa-miR-431*, hsa-miR-378i, hsa-miR-422a, hsa-miR-331-5p, hsa-miR-378*, hsa-miR-148b, hsa-miR-601, hsa-miR-4288, hsa-miR-596, hsa-miR-10a, hsa-miR-200c*, hsa-miR-378b, hsa-miR-378f, hsa-miR-4257, hsa-miR-370, hsa-miR-1237, hsa-miR-3942-3p

3) hsa-miR-211, hsa-miR-29b, hsa-miR-4324, hsa-miR-143*, hsa-miR-1287, hsa-miR-4708-5p, hsa-miR-195*, hsa-miR-508-5p, hsa-miR-204, hsa-miR-96, hsa-let-7b*, hsa-miR-935, hsa-miR-675*, hsa-miR-149, hsa-miR-30a*, hsa-miR-1181, hsa-miR-506, hsa-miR-23c, hsa-miR-127-5p, hsa-miR-491-5p

4) hsa-miR-30b, hsa-miR-221, hsa-miR-141, hsa-miR-339-3p, hsa-miR-100, hsa-miR-331, hsa-miR-598, hsa-miR-24, hsa-miR-23a, hsa-miR-99a, hsa-miR-152, hsa-miR-423-5p, hsa-miR-34c, hsa-miR-27a, hsa-miR-30c, hsa-miR-199a, hsa-miR-125a-5p, mmu-miR-491, hsa-miR-489, hsa-miR-337-5p, hsa-miR-149, hsa-miR-127, hsa-miR-375, hsa-miR-101, hsa-miR-296, mmu-miR-379, hsa-miR-574-3p, hsa-miR-487b, mmu-miR-499, hsa-miR-95, hsa-miR-885-5p, hsa-miR-486-3p, hsa-miR-1, hsa-miR-133b, hsa-miR-133a.

a) hsa-miR-133a, hsa-miR-133b, hsa-miR-1, hsa-miR-299-5p, hsa-miR-486-3p, hsa-miR-381, hsa-miR-4324, hsa-miR-154, hsa-miR-1247, hsa-miR-1287, hsa-miR-376c, hsa-miR-195*, hsa-miR-4269, hsa-miR-1296, hsa-miR-34c-3p, hsa-miR-204, hsa-miR-504, hsa-miR-30c-1*, hsa-miR-615-3p, hsa-miR-505, hsa-miR-508-5p, hsa-miR-409-5p, hsa-miR-433, hsa-miR-375, hsa-miR-935, hsa-miR-128, hsa-miR-331-5p, hsa-miR-584, hsa-miR-30a*, hsa-miR-370, hsa-miR-148a*, hsa-miR-489, hsa-miR-30e*, hsa-miR-337-5p, hsa-miR-1181, hsa-miR-1237, hsa-miR-485-5p, hsa-miR-23c, hsa-miR-148b, hsa-miR-378g, hsa-miR-1238, hsa-miR-181a-2*, hsa-miR-187, hsa-miR-378d, hsa-miR-181d, hsa-miR-98, hsa-miR-4685-3p, hsa-miR-23b*

b) hsa-miR-331, hsa-miR-598, hsa-miR-24, hsa-miR-23a, hsa-miR-99a, hsa-miR-152, hsa-miR-423-5p, hsa-miR-34c, hsa-miR-27a, hsa-miR-30c, hsa-miR-125a-5p, mmu-miR-491, hsa-miR-127, hsa-miR-375, hsa-miR-101, hsa-miR-296, mmu-miR-379, hsa-miR-574-3p, hsa-miR-487b, mmu-miR-499, hsa-miR-95, hsa-miR-885-5p, hsa-miR-486-3p, hsa-miR-1, hsa-miR-133b, hsa-miR-133a

c) hsa-miR-133a, hsa-miR-133b, hsa-miR-1, hsa-miR-299-5p, hsa-miR-486-3p, hsa-miR-381, hsa-miR-4324, hsa-miR-154, hsa-miR-1247, hsa-miR-885-5p, hsa-miR-1287, hsa-miR-95, hsa-miR-376c, hsa-miR-195*, hsa-miR-4269, mmu-miR-499, hsa-miR-1296, hsa-miR-34c-3p, hsa-miR-204, hsa-miR-504, hsa-miR-30c-1*, hsa-miR-487b, hsa-miR-615-3p, hsa-miR-505, hsa-miR-508-5p, hsa-miR-409-5p, hsa-miR-433, hsa-miR-375, hsa-miR-935, hsa-miR-128, hsa-miR-331-5p, hsa-miR-584, hsa-miR-30a*, hsa-miR-370, hsa-miR-148a*, hsa-miR-489, hsa-miR-30e*, hsa-miR-337-5p, hsa-miR-1181, hsa-miR-1237, hsa-miR-485-5p, hsa-miR-23c, hsa-miR-491-5p, hsa-miR-148b, hsa-miR-378g, hsa-miR-125a-5p, hsa-miR-1238, hsa-miR-181a-2*, hsa-miR-187, hsa-miR-30c, hsa-miR-27a, hsa-miR-99a, hsa-miR-378d, hsa-miR-181d, hsa-miR-98, hsa-miR-4685-3p, hsa-miR-598, hsa-miR-23b*

d) hsa-miR-133a, hsa-miR-133b, hsa-miR-1, hsa-miR-486-3p, hsa-miR-34c-3p, hsa-miR-375.

7. Méthode selon l'une des revendications 1 à 6, dans laquelle l'échantillon est une biopsie de peau prélevée au niveau des zones de la peau atteintes.

8. Méthode pour suivre ou déterminer l'efficacité d'un traitement de la rosacée de type II chez un sujet comprenant la détermination dans un échantillon de derme et/ou épiderme dudit sujet de l'expression d'au moins une combinaison de sept microARNs suivants : hsa-miR-3201, hsa-miR-4423-3p, hsa-miR-3128, hsa-miR-3163, hsa-miR-606, hsa-miR-4776-5p, et hsa-miR-635, et la diminution de l'expression ou activité des microARNs à l'issue ou pendant le traitement indiquant l'efficacité du traitement.

9. Méthode selon la revendication 8, dans laquelle l'échantillon est une biopsie de peau prélevée au niveau des zones de la peau atteintes.

**Patentansprüche**

1. Verfahren zur Diagnose einer Rosacea vom Typ II bei einem Subjekt, umfassend Ermittlung, in einer Lederhaut- und/oder Epidermisprobe des genannten Subjekts, der Expression wenigstens der Kombination der folgenden sieben MikroRNAs: hsa-miR-3201, hsa-miR-4423-3p, hsa-miR-3128, hsa-miR-3163, hsa-miR-606, hsa-miR-4776-5p, und hsa-miR-635, wobei ein Expression der genannten MikroRNAs der genannten Probe, der höher als der von Kontrollproben ist, angibt, dass das Subjekt an besagter Rosacea leidet.

2. Verfahren nach Anspruch 1, umfassend zusätzlicher Ermittlung der Expression einer oder mehrerer MikroRNAs aus der Gruppe

| hsa-miR-133a | hsa-miR-3201 |
|---|---|
| hsa-miR-133b | hsa-miR-4423-3p |
| hsa-miR-1 | hsa-miR-3128 |
| hsa-miR-299-5p | hsa-miR-155 |
| hsa-miR-486-3p | hsa-miR-3163 |
| hsa-miR-381 | hsa-miR-146b-5p |
| hsa-miR-4324 | hsa-miR-606 |

(fortgesetzt)

| | |
|---|---|
| hsa-miR-154 | hsa-miR-150 |
| hsa-miR-1247 | hsa-miR-4776-5p |
| hsa-miR-1287 | hsa-miR-3124-5p |
| hsa-miR-376c | hsa-miR-4741 |
| hsa-miR-195* | hsa-miR-635 |
| hsa-miR-4269 | hsa-miR-4668-5p |
| hsa-miR-1296 | hsa-miR-4763-3p |
| hsa-miR-34c-3p | hsa-miR-4417 |
| hsa-miR-204 | hsa-miR-1911* |
| hsa-miR-504 | hsa-miR-4734 |
| hsa-miR-30c-1* | hsa-miR-4530 |
| hsa-miR-615-3p | hsa-miR-4260 |
| hsa-miR-505 | hsa-miR-3185 |
| hsa-miR-508-5p | hsa-miR-4707-5p |
| hsa-miR-409-5p | hsa-miR-4745-5p |
| hsa-miR-433 | hsa-miR-4659a-3p |
| hsa-miR-375 | hsa-miR-1469 |
| hsa-miR-935 | hsa-miR-4695-5p |
| hsa-miR-128 | hsa-miR-4674 |
| hsa-miR-331-5p | hsa-miR-4687-3p |
| hsa-miR-584 | hsa-miR-4739 |
| hsa-miR-30a* | hsa-miR-1915 |
| hsa-miR-370 | hsa-miR-216b |
| hsa-miR-148a* | hsa-miR-548a-3p |
| hsa-miR-489 | |
| hsa-miR-30e* | |
| hsa-miR-337-5p | |
| hsa-miR-1181 | |
| hsa-miR-1237 | |
| hsa-miR-485-5p | |
| hsa-miR-23c | |
| hsa-miR-148b | |
| hsa-miR-378g | |
| hsa-miR-1238 | |
| hsa-miR-181a-2* | |
| hsa-miR-187 | |
| hsa-miR-378d | |
| hsa-miR-181d | |
| hsa-miR-98 | |
| hsa-miR-4685-3p | |
| hsa-miR-23b* | |

3. Verfahren nach Anspruch 1 oder 2, umfassend zusätzlicher Ermittlung der Expression einer oder mehrerer MikroRNAs aus einer der folgenden Gruppen

a) hsa-miR-133a, hsa-miR-133b, hsa-miR-1, hsa-miR-299-5p, hsa-miR-486-3p, hsa-miR-381, hsa-miR-4324, hsa-miR-154, hsa-miR-1247, hsa-miR-1287, hsa-miR-376c, hsa-miR-195*, hsa-miR-4269, hsa-miR-1296, hsa-miR-34c-3p, hsa-miR-204, hsa-miR-504, hsa-miR-30c-1*, hsa-miR-615-3p, hsa-miR-505, hsa-miR-508-5p, hsa-miR-409-5p, hsa-miR-433, hsa-miR-375, hsa-miR-935, hsa-miR-128, hsa-miR-331-5p, hsa-miR-584, hsa-miR-30a*, hsa-miR-370, hsa-miR-148a*, hsa-miR-489, hsa-miR-30e*, hsa-miR-337-5p, hsa-miR-1181, hsa-miR-1237, hsa-miR-485-5p, hsa-miR-23c, hsa-miR-148b, hsa-miR-378g, hsa-miR-1238, hsa-miR-181a-2*, hsa-miR-187, hsa-miR-378d, hsa-miR-181d, hsa-miR-98, hsa-miR-4685-3p, hsa-miR-23b*, hsa-miR-3201, hsa-miR-4423-3p, hsa-miR-3128, hsa-miR-155, hsa-miR-3163, hsa-miR-146b-5p, hsa-miR-606, hsa-miR-150, hsa-miR-4776-5p, hsa-miR-3124-5p, hsa-miR-4741, hsa-miR-635, hsa-miR-4668-5p, hsa-miR-4763-3p, hsa-miR-4417, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

b) hsa-miR-223, hsa-miR-142-3p, hsa-miR-146b, hsa-miR-21, hsa-miR-331, hsa-miR-598, hsa-miR-24, hsa-miR-23a, hsa-miR-99a, hsa-miR-152, hsa-miR-423-5p, hsa-miR-34c, hsa-miR-27a, hsa-miR-30c, hsa-miR-125a-5p, mmu-miR-491, hsa-miR-127, hsa-miR-375, hsa-miR-101, hsa-miR-296, mmu-miR-379, hsa-miR-574-3p, hsa-miR-487b, mmu-miR-499, hsa-miR-95, hsa-miR-885-5p, hsa-miR-486-3p, hsa-miR-1, hsa-miR-133b, hsa-miR-133a

c) hsa-miR-133a, hsa-miR-133b, hsa-miR-1, hsa-miR-299-5p, hsa-miR-486-3p, hsa-miR-381, hsa-miR-4324, hsa-miR-154, hsa-miR-1247, hsa-miR-885-5p, hsa-miR-1287, hsa-miR-95, hsa-miR-376c, hsa-miR-195*, hsa-miR-4269, mmu-miR-499, hsa-miR-1296, hsa-miR-34c-3p, hsa-miR-204, hsa-miR-504, hsa-miR-30c-1*, hsa-miR-487b, hsa-miR-615-3p, hsa-miR-505, hsa-miR-508-5p, hsa-miR-409-5p, hsa-miR-433, hsa-miR-375, hsa-miR-935, hsa-miR-128, hsa-miR-331-5p, hsa-miR-584, hsa-miR-30a*, hsa-miR-370, hsa-miR-148a*, hsa-miR-489, hsa-miR-30e*, hsa-miR-337-5p, hsa-miR-1181, hsa-miR-1237, hsa-miR-485-5p, hsa-miR-23c, hsa-miR-491-5p, hsa-miR-148b, hsa-miR-378g, hsa-miR-125a-5p, hsa-miR-1238, hsa-miR-181 a-2*, hsa-miR-187, hsa-miR-30c, hsa-miR-27a, hsa-miR-99a, hsa-miR-378d, hsa-miR-181d, hsa-miR-98, hsa-miR-4685-3p, hsa-miR-598, hsa-miR-23b*, hsa-miR-3201, hsa-miR-4423-3p, hsa-miR-223, hsa-miR-3128, hsa-miR-155, hsa-miR-3163, hsa-miR-146b-5p, hsa-miR-606, hsa-miR-150, hsa-miR-4776-5p, hsa-miR-142-3p, hsa-miR-21, hsa-miR-3124-5p, hsa-miR-4741, hsa-miR-635, hsa-miR-4668-5p, hsa-miR-4763-3p, hsa-miR-4417, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

d) hsa-miR-3201, hsa-miR-4423-3p, hsa-miR-3128, hsa-miR-3163, hsa-miR-606, hsa-miR-4776-5p, hsa-miR-635, hsa-miR-3124-5p, hsa-miR-4741, hsa-miR-4668-5p, hsa-miR-4763-3p, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p.

4. Verfahren nach einem der Ansprüche 1 bis 3, umfassend zusätzlicher Ermittlung der Expression einer oder mehrerer MikroRNAs ausgewählt aus hsa-miR-133a, hsa-miR-133b, hsa-miR-1, hsa-miR-486-3p, hsa-miR-34c-3p, hsa-miR-375, und hsa-miR-146b-5p.

5. Verfahren nach Anspruch 1, umfassend zusätzlicher Ermittlung der Expression einer oder mehrerer MikroRNAs aus einer der folgenden Gruppen

1) hsa-miR-4758-5p, hsa-miR-4665-5p, hsa-miR-149*, hsa-miR-548ac, hsa-miR-4507, hsa-miR-548a-3p, hsa-miR-4289, hsa-miR-4727-3p, hsa-miR-4468, hsa-miR-4463, hsa-miR-2861, hsa-miR-4773, hsa-miR-1825, hsa-miR-4651, hsa-miR-216b, hsa-miR-4689, hsa-miR-3152-3p, hsa-miR-4270, hsa-miR-1915, hsa-miR-4739, hsa-miR-4772-5p, hsa-miR-4687-3p, hsa-miR-4674, hsa-miR-4695-5p, hsa-miR-4439, hsa-miR-129-3p, hsa-miR-1469, hsa-miR-4659a-3p, hsa-miR-4799-3p, hsa-miR-4657, hsa-miR-4655-5p, hsa-miR-4745-5p, hsa-miR-4662b, hsa-miR-4707-5p, hsa-miR-3185, hsa-miR-4260, hsa-miR-4530, hsa-miR-4734, hsa-miR-1911*, hsa-miR-4417, hsa-miR-4763-3p, hsa-miR-4668-5p, hsa-miR-1281, hsa-miR-635, hsa-miR-4741, hsa-miR-3124-5p, hsa-miR-146b-3p, hsa-miR-4529-3p, hsa-miR-4776-5p, hsa-miR-150, hsa-miR-3927, hsa-miR-606, hsa-miR-146b-5p, hsa-miR-3163, hsa-miR-155, hsa-miR-371b-5p, hsa-miR-3128, hsa-miR-4423-3p, hsa-miR-335, hsa-miR-3201, hsa-miR-184

2) hsa-miR-1269b, hsa-miR-4741, hsa-miR-4763-3p, hsa-miR-4704-5p, hsa-miR-1911*, hsa-miR-3621, hsa-miR-3612, hsa-miR-4436b-5p, hsa-miR-4734, hsa-miR-3185, hsa-miR-3910, hsa-miR-4727-3p, hsa-miR-155, hsa-miR-155*, hsa-miR-4529-3p, hsa-miR-3927, hsa-miR-3201, hsa-miR-4423-3p, hsa-miR-124, hsa-miR-184;

3) hsa-miR-4776-5p, hsa-miR-601, hsa-miR-4530, hsa-miR-4773, hsa-miR-4717-3p, hsa-miR-4657, hsa-miR-4289, hsa-miR-4417, hsa-miR-4445*, hsa-miR-150, hsa-miR-146b-5p, hsa-miR-3175, hsa-miR-4646-5p, hsa-miR-3163, hsa-miR-4423-3p, hsa-miR-3128, hsa-miR-1244, hsa-miR-21*, hsa-miR-3201, hsa-miR-335

4) hsa-miR-223, hsa-miR-142-3p, hsa-miR-146b, hsa-miR-21, hsa-miR-155

a) hsa-miR-3201, hsa-miR-4423-3p, hsa-miR-3128, hsa-miR-155, hsa-miR-3163, hsa-miR-146b-5p, hsa-miR-606, hsa-miR-150, hsa-miR-4776-5p, hsa-miR-3124-5p, hsa-miR-4741, hsa-miR-635, hsa-miR-4668-5p, hsa-miR-4763-3p, hsa-miR-4417, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

b) hsa-miR-223, hsa-miR-142-3p, hsa-miR-146b, hsa-miR-21

c) hsa-miR-3201, hsa-miR-4423-3p, hsa-miR-223, hsa-miR-3128, hsa-miR-155, hsa-miR-3163, hsa-miR-146b-5p, hsa-miR-606, hsa-miR-150, hsa-miR-4776-5p, hsa-miR-142-3p, hsa-miR-21, hsa-miR-3124-5p, hsa-miR-4741, hsa-miR-635, hsa-miR-4668-5p, hsa-miR-4763-3p, hsa-miR-4417, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

d) hsa-miR-3201, hsa-miR-4423-3p, hsa-miR-3128, hsa-miR-3163, hsa-miR-606, hsa-miR-4776-5p, hsa-miR-635, hsa-miR-3124-5p, hsa-miR-4741, hsa-miR-4668-5p, hsa-miR-4763-3p, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

e) hsa-miR-146b-5p.

6. Verfahren nach Anspruch 1, umfassend zusätzlicher Ermittlung, in einer Probe des genannten Subjekts, der Expression einer oder mehrerer MikroRNAs aus der Gruppe

1) hsa-miR-133a, hsa-miR-206, hsa-miR-133b, hsa-miR-1, hsa-miR-299-5p, hsa-miR-486-3p, hsa-miR-381, hsa-miR-4324, hsa-miR-154, hsa-miR-1247, hsa-miR-1287, hsa-miR-376c, hsa-miR-195*, hsa-miR-411, hsa-miR-4269, hsa-miR-328, hsa-miR-1296, hsa-miR-34c-3p, hsa-miR-204, hsa-miR-504, hsa-miR-30c-1*, hsa-miR-615-3p, hsa-miR-654-3p, hsa-miR-505, hsa-miR-508-5p, hsa-miR-409-5p, hsa-miR-338-5p, hsa-miR-433, hsa-miR-375, hsa-miR-935, hsa-let-7d*, hsa-miR-99a*, hsa-miR-128, hsa-miR-1290, hsa-miR-331-5p, hsa-miR-4730, hsa-miR-29c*, hsa-miR-455-5p, hsa-miR-377*, hsa-miR-378e, hsa-miR-584, hsa-miR-96, hsa-miR-30a*, hsa-miR-143*, hsa-miR-370, hsa-miR-148a*, hsa-miR-489, hsa-miR-506, hsa-let-7e*, hsa-miR-30e*, hsa-miR-4787-3p, hsa-let-7b*, hsa-miR-181c*, hsa-miR-513a-5p, hsa-miR-92a-1*, hsa-miR-337-5p, hsa-miR-1181, hsa-miR-1237, hsa-miR-3620, hsa-miR-485-5p, hsa-miR-23c, hsa-miR-18a*, hsa-miR-10a, hsa-miR-139-3p, hsa-miR-493, hsa-miR-148b, hsa-miR-550a, hsa-miR-3147, hsa-miR-378g, hsa-miR-675*, hsa-miR-1238, hsa-miR-542-5p, hsa-miR-30c-2*, hsa-miR-125a-3p, hsa-miR-181a-2*, hsa-miR-1292, hsa-miR-187, hsa-miR-378*, hsa-miR-493*, hsa-miR-495, hsa-miR-557, hsa-miR-3909, hsa-miR-378d, hsa-miR-491-5p, hsa-miR-181d, hsa-miR-671-3p, hsa-miR-513c, hsa-miR-487b, hsa-miR-378b, hsa-miR-885-5p, hsa-miR-98, hsa-miR-29b-2*, hsa-miR-4685-3p, hsa-miR-3605-3p, hsa-miR-24-1*, hsa-miR-4649-3p, hsa-miR-3180-5p, hsa-miR-149, hsa-miR-23b*

2) hsa-miR-206, hsa-miR-133b, hsa-miR-133a, hsa-miR-1, hsa-miR-486-3p, hsa-miR-299-5p, hsa-miR-1247, hsa-miR-381, hsa-miR-154, hsa-miR-433, hsa-miR-1244, hsa-miR-4485, hsa-miR-378e, hsa-miR-505, hsa-miR-376c, hsa-miR-1296, hsa-miR-29b-1*, hsa-miR-409-5p, hsa-miR-29c*, hsa-miR-34c-3p, hsa-miR-4646-5p, hsa-miR-99a*, hsa-miR-378g, hsa-miR-504, hsa-miR-584, hsa-miR-128, hsa-miR-30c-1*, hsa-miR-665, hsa-miR-4269, hsa-miR-493*, hsa-miR-328, hsa-miR-550a, hsa-miR-375, hsa-miR-493, hsa-miR-378d, hsa-miR-148a*, hsa-miR-615-3p, hsa-miR-431*, hsa-miR-378i, hsa-miR-422a, hsa-miR-331-5p, hsa-miR-378*, hsa-miR-148b, hsa-miR-601, hsa-miR-4288, hsa-miR-596, hsa-miR-10a, hsa-miR-200c*, hsa-miR-378b, hsa-miR-378f, hsa-miR-4257, hsa-miR-370, hsa-miR-1237, hsa-miR-3942-3p

3) hsa-miR-211, hsa-miR-29b, hsa-miR-4324, hsa-miR-143*, hsa-miR-1287, hsa-miR-4708-5p, hsa-miR-195*, hsa-miR-508-5p, hsa-miR-204, hsa-miR-96, hsa-let-7b*, hsa-miR-935, hsa-miR-675*, hsa-miR-149, hsa-miR-30a*, hsa-miR-1181, hsa-miR-506, hsa-miR-23c, hsa-miR-127-5p, hsa-miR-491-5p

4) hsa-miR-30b, hsa-miR-221, hsa-miR-141, hsa-miR-339-3p, hsa-miR-100, hsa-miR-331, hsa-miR-598, hsa-miR-24, hsa-miR-23a, hsa-miR-99a, hsa-miR-152, hsa-miR-423-5p, hsa-miR-34c, hsa-miR-27a, hsa-miR-30c, hsa-miR-199a, hsa-miR-125a-5p, mmu-miR-491, hsa-miR-489, hsa-miR-337-5p, hsa-miR-149, hsa-miR-127, hsa-miR-375, hsa-miR-101, hsa-miR-296, mmu-miR-379, hsa-miR-574-3p, hsa-miR-487b, mmu-miR-499, hsa-miR-95, hsa-miR-885-5p, hsa-miR-486-3p, hsa-miR-1, hsa-miR-133b, hsa-miR-133a.

a) hsa-miR-133a, hsa-miR-133b, hsa-miR-1, hsa-miR-299-5p, hsa-miR-486-3p, hsa-miR-381, hsa-miR-4324, hsa-miR-154, hsa-miR-1247, hsa-miR-1287, hsa-miR-376c, hsa-miR-195*, hsa-miR-4269, hsa-miR-1296, hsa-miR-34c-3p, hsa-miR-204, hsa-miR-504, hsa-miR-30c-1*, hsa-miR-615-3p, hsa-miR-505, hsa-miR-508-5p, hsa-miR-409-5p, hsa-miR-433, hsa-miR-375, hsa-miR-935, hsa-miR-128, hsa-miR-331-5p, hsa-miR-584, hsa-miR-30a*, hsa-miR-370, hsa-miR-148a*, hsa-miR-489, hsa-miR-30e*, hsa-miR-337-5p,

hsa-miR-1181, hsa-miR-1237, hsa-miR-485-5p, hsa-miR-23c, hsa-miR-148b, hsa-miR-378g, hsa-miR-1238, hsa-miR-181a-2*, hsa-miR-187, hsa-miR-378d, hsa-miR-181d, hsa-miR-98, hsa-miR-4685-3p, hsa-miR-23b*

b) hsa-miR-331, hsa-miR-598, hsa-miR-24, hsa-miR-23a, hsa-miR-99a, hsa-miR-152, hsa-miR-423-5p, hsa-miR-34c, hsa-miR-27a, hsa-miR-30c, hsa-miR-125a-5p, mmu-miR-491, hsa-miR-127, hsa-miR-375, hsa-miR-101, hsa-miR-296, mmu-miR-379, hsa-miR-574-3p, hsa-miR-487b, mmu-miR-499, hsa-miR-95, hsa-miR-885-5p, hsa-miR-486-3p, hsa-miR-1, hsa-miR-133b, hsa-miR-133a

c) hsa-miR-133a, hsa-miR-133b, hsa-miR-1, hsa-miR-299-5p, hsa-miR-486-3p, hsa-miR-381, hsa-miR-4324, hsa-miR-154, hsa-miR-1247, hsa-miR-885-5p, hsa-miR-1287, hsa-miR-95, hsa-miR-376c, hsa-miR-195*, hsa-miR-4269, mmu-miR-499, hsa-miR-1296, hsa-miR-34c-3p, hsa-miR-204, hsa-miR-504, hsa-miR-30c-1*, hsa-miR-487b, hsa-miR-615-3p, hsa-miR-505, hsa-miR-508-5p, hsa-miR-409-5p, hsa-miR-433, hsa-miR-375, hsa-miR-935, hsa-miR-128, hsa-miR-331-5p, hsa-miR-584, hsa-miR-30a*, hsa-miR-370, hsa-miR-148a*, hsa-miR-489, hsa-miR-30e*, hsa-miR-337-5p, hsa-miR-1181, hsa-miR-1237, hsa-miR-485-5p, hsa-miR-23c, hsa-miR-491-5p, hsa-miR-148b, hsa-miR-378g, hsa-miR-125a-5p, hsa-miR-1238, hsa-miR-181a-2*, hsa-miR-187, hsa-miR-30c, hsa-miR-27a, hsa-miR-99a, hsa-miR-378d, hsa-miR-181d, hsa-miR-98, hsa-miR-4685-3p, hsa-miR-598, hsa-miR-23b*

d) hsa-miR-133a, hsa-miR-133b, hsa-miR-1, hsa-miR-486-3p, hsa-miR-34c-3p, hsa-miR-375.

7. Verfahren nach einem der Ansprüche 1 bis 6, wobei die Probe eine Biopsie von Haut ist, die in den Bereichen der erkrankten Haut entnommen wurde.

8. Verfahren, um die Effektivität einer Behandlung der Rosacea vom Typ II bei einem Subjekt zu verfolgen oder zu bestimmen, umfassend Ermittlung, in einer Lederhaut- und/oder Epidermisprobe des genannten Subjekts, der Expression wenigstens einer Kombination der folgenden sieben MikroRNAs: hsa-miR-3201; hsa-miR-4423-3p, hsa-miR-3128; hsa-miR-3163; hsa-miR-606; hsa-miR-4776-5p und hsa-miR-635, wobei die Verringerung der Expression oder der Aktivität der MikroRNAs am Ende oder während der Behandlung die Effektivität der Behandlung angibt.

9. Verfahren nach Anspruch 8, wobei die Probe eine Biopsie von Haut ist, die in den Bereichen der erkrankten Haut entnommen wurde.

## Claims

1. A method for diagnosing a type II rosacea in a subject, comprising determining in a dermal and/or epidermal sample from said subject the expression of at least the combination of the following seven microRNAs: hsa-miR-3201, hsa-miR-4423-3p, hsa-miR-3128, hsa-miR-3163, hsa-miR-606, hsa-miR-4776-5p, and hsa-miR-635, and wherein an expression of said microRNAs form said sample being higher than that of control samples indicates that the subject suffers from said rosacea.

2. The method of claim 1, comprising further determining the expression of one or more microRNAs selected from:

| | |
|---|---|
| hsa-miR-133a | hsa-miR-3201 |
| hsa-miR-133b | hsa-miR-4423-3p |
| hsa-miR-1 | hsa-miR-3128 |
| hsa-miR-299-5p | hsa-miR-155 |
| hsa-miR-486-3p | hsa-miR-3163 |
| hsa-miR-381 | hsa-miR-146b-5p |
| hsa-miR-4324 | hsa-miR-606 |
| hsa-miR-154 | hsa-miR-150 |
| hsa-miR-1247 | hsa-miR-4776-5p |
| hsa-miR-1287 | hsa-miR-3124-5p |
| hsa-miR-376c | hsa-miR-4741 |
| hsa-miR-195* | hsa-miR-635 |
| hsa-miR-4269 | hsa-miR-4668-5p |
| hsa-miR-1296 | hsa-miR-4763-3p |

(continued)

| | |
|---|---|
| hsa-miR-34c-3p | hsa-miR-4417 |
| hsa-miR-204 | hsa-miR-1911* |
| hsa-miR-504 | hsa-miR-4734 |
| hsa-miR-30c-1* | hsa-miR-4530 |
| hsa-miR-615-3p | hsa-miR-4260 |
| hsa-miR-505 | hsa-miR-3185 |
| hsa-miR-508-5p | hsa-miR-4707-5p |
| hsa-miR-409-5p | hsa-miR-4745-5p |
| hsa-miR-433 | hsa-miR-4659a-3p |
| hsa-miR-375 | hsa-miR-1469 |
| hsa-miR-935 | hsa-miR-4695-5p |
| hsa-miR-128 | hsa-miR-4674 |
| hsa-miR-331-5p | hsa-miR-4687-3p |
| hsa-miR-584 | hsa-miR-4739 |
| hsa-miR-30a* | hsa-miR-1915 |
| hsa-miR-370 | hsa-miR-216b |
| hsa-miR-148a* | hsa-miR-548a-3p |
| hsa-miR-489 | |
| hsa-miR-30e* | |
| hsa-miR-337-5p | |
| hsa-miR-1181 | |
| hsa-miR-1237 | |
| hsa-miR-485-5p | |
| hsa-miR-23c | |
| hsa-miR-148b | |
| hsa-miR-378g | |
| hsa-miR-1238 | |
| hsa-miR-181a-2* | |
| hsa-miR-187 | |
| hsa-miR-378d | |
| hsa-miR-181d | |
| hsa-miR-98 | |
| hsa-miR-4685-3p | |
| hsa-miR-23b* | |

3. Method according to claim 1 or 2, comprising further determining the expression of one or more microRNAs selected from one of the groups

a) hsa-miR-133a, hsa-miR-133b, hsa-miR-1, hsa-miR-299-5p, hsa-miR-486-3p, hsa-miR-381, hsa-miR-4324, hsa-miR-154, hsa-miR-1247, hsa-miR-1287, hsa-miR-376c, hsa-miR-195*, hsa-miR-4269, hsa-miR-1296, hsa-miR-34c-3p, hsa-miR-204, hsa-miR-504, hsa-miR-30c-1*, hsa-miR-615-3p, hsa-miR-505, hsa-miR-508-5p, hsa-miR-409-5p, hsa-miR-433, hsa-miR-375, hsa-miR-935, hsa-miR-128, hsa-miR-331-5p, hsa-miR-584, hsa-miR-30a*, hsa-miR-370, hsa-miR-148a*, hsa-miR-489, hsa-miR-30e*, hsa-miR-337-5p, hsa-miR-1181, hsa-miR-1237, hsa-miR-485-5p, hsa-miR-23c, hsa-miR-148b, hsa-miR-378g, hsa-miR-1238, hsa-miR-181a-2*, hsa-miR-187, hsa-miR-378d, hsa-miR-181d, hsa-miR-98, hsa-miR-4685-3p, hsa-miR-23b*, hsa-miR-3201, hsa-miR-4423-3p, hsa-miR-3128, hsa-miR-155, hsa-miR-3163, hsa-miR-146b-5p, hsa-miR-606, hsa-miR-150, hsa-miR-4776-5p, hsa-miR-3124-5p, hsa-miR-4741, hsa-miR-635, hsa-miR-4668-5p, hsa-miR-4763-3p, hsa-miR-4417, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

b) hsa-miR-223, hsa-miR-142-3p, hsa-miR-146b, hsa-miR-21, hsa-miR-331, hsa-miR-598, hsa-miR-24, hsa-miR-23a, hsa-miR-99a, hsa-miR-152, hsa-miR-423-5p, hsa-miR-34c, hsa-miR-27a, hsa-miR-30c, hsa-miR-125a-5p, mmu-miR-491, hsa-miR-127, hsa-miR-375, hsa-miR-101, hsa-miR-296, mmu-miR-379, hsa-miR-574-3p, hsa-miR-487b, mmu-miR-499, hsa-miR-95, hsa-miR-885-5p, hsa-miR-486-3p, hsa-miR-1, hsa-miR-133b, hsa-miR-133a

c) hsa-miR-133a, hsa-miR-133b, hsa-miR-1, hsa-miR-299-5p, hsa-miR-486-3p, hsa-miR-381, hsa-miR-4324, hsa-miR-154, hsa-miR-1247, hsa-miR-885-5p, hsa-miR-1287, hsa-miR-95, hsa-miR-376c, hsa-miR-195*, hsa-miR-4269, mmu-miR-499, hsa-miR-1296, hsa-miR-34c-3p, hsa-miR-204, hsa-miR-504, hsa-miR-30c-1*, hsa-miR-487b, hsa-miR-615-3p, hsa-miR-505, hsa-miR-508-5p, hsa-miR-409-5p, hsa-miR-433, hsa-miR-375, hsa-miR-935, hsa-miR-128, hsa-miR-331-5p, hsa-miR-584, hsa-miR-30a*, hsa-miR-370, hsa-miR-148a*, hsa-miR-489, hsa-miR-30e*, hsa-miR-337-5p, hsa-miR-1181, hsa-miR-1237, hsa-miR-485-5p, hsa-miR-23c, hsa-miR-491-5p, hsa-miR-148b, hsa-miR-378g, hsa-miR-125a-5p, hsa-miR-1238, hsa-miR-181a-2*, hsa-miR-187, hsa-miR-30c, hsa-miR-27a, hsa-miR-99a, hsa-miR-378d, hsa-miR-181d, hsa-miR-98, hsa-miR-4685-3p, hsa-miR-598, hsa-miR-23b*, hsa-miR-3201, hsa-miR-4423-3p, hsa-miR-223, hsa-miR-3128, hsa-miR-155, hsa-miR-3163, hsa-miR-146b-5p, hsa-miR-606, hsa-miR-150, hsa-miR-4776-5p, hsa-miR-142-3p, hsa-miR-21, hsa-miR-3124-5p, hsa-miR-4741, hsa-miR-635, hsa-miR-4668-5p, hsa-miR-4763-3p, hsa-miR-4417, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

d) hsa-miR-3201, hsa-miR-4423-3p, hsa-miR-3128, hsa-miR-3163, hsa-miR-606, hsa-miR-4776-5p, hsa-miR-635, hsa-miR-3124-5p, hsa-miR-4741, hsa-miR-4668-5p, hsa-miR-4763-3p, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p.

4. Method according to one of claims 1 to 3, comprising further determining the expression of one or more microRNAs selected from hsa-miR-133a, hsa-miR-133b, hsa-miR-1, hsa-miR-486-3p, hsa-miR-34c-3p, hsa-miR-375, and hsa-miR-146b-5p.

5. The method of claim 1, comprising further determining the expression of one or more microRNAs selected from one of the groups consisting of

1) hsa-miR-4758-5p, hsa-miR-4665-5p, hsa-miR-149*, hsa-miR-548ac, hsa-miR-4507, hsa-miR-548a-3p, hsa-miR-4289, hsa-miR-4727-3p, hsa-miR-4468, hsa-miR-4463, hsa-miR-2861, hsa-miR-4773, hsa-miR-1825, hsa-miR-4651, hsa-miR-216b, hsa-miR-4689, hsa-miR-3152-3p, hsa-miR-4270, hsa-miR-1915, hsa-miR-4739, hsa-miR-4772-5p, hsa-miR-4687-3p, hsa-miR-4674, hsa-miR-4695-5p, hsa-miR-4439, hsa-miR-129-3p, hsa-miR-1469, hsa-miR-4659a-3p, hsa-miR-4799-3p, hsa-miR-4657, hsa-miR-4655-5p, hsa-miR-4745-5p, hsa-miR-4662b, hsa-miR-4707-5p, hsa-miR-3185, hsa-miR-4260, hsa-miR-4530, hsa-miR-4734, hsa-miR-1911*, hsa-miR-4417, hsa-miR-4763-3p, hsa-miR-4668-5p, hsa-miR-1281, hsa-miR-635, hsa-miR-4741, hsa-miR-3124-5p, hsa-miR-146b-3p, hsa-miR-4529-3p, hsa-miR-4776-5p, hsa-miR-150, hsa-miR-3927, hsa-miR-606, hsa-miR-146b-5p, hsa-miR-3163, hsa-miR-155, hsa-miR-371b-5p, hsa-miR-3128, hsa-miR-4423-3p, hsa-miR-335, hsa-miR-3201, hsa-miR-184

2) hsa-miR-1269b, hsa-miR-4741, hsa-miR-4763-3p, hsa-miR-4704-5p, hsa-miR-1911*, hsa-miR-3621, hsa-miR-3612, hsa-miR-4436b-5p, hsa-miR-4734, hsa-miR-3185, hsa-miR-3910, hsa-miR-4727-3p, hsa-miR-155, hsa-miR-155*, hsa-miR-4529-3p, hsa-miR-3927, hsa-miR-3201, hsa-miR-4423-3p, hsa-miR-124, hsa-miR-184;

3) hsa-miR-4776-5p, hsa-miR-601, hsa-miR-4530, hsa-miR-4773, hsa-miR-4717-3p, hsa-miR-4657, hsa-miR-4289, hsa-miR-4417, hsa-miR-4445*, hsa-miR-150, hsa-miR-146b-5p, hsa-miR-3175, hsa-miR-4646-5p, hsa-miR-3163, hsa-miR-4423-3p, hsa-miR-3128, hsa-miR-1244, hsa-miR-21*, hsa-miR-3201, hsa-miR-335

4) hsa-miR-223, hsa-miR-142-3p, hsa-miR-146b, hsa-miR-21, hsa-miR-155

a) hsa-miR-3201, hsa-miR-4423-3p, hsa-miR-3128, hsa-miR-155, hsa-miR-3163, hsa-miR-146b-5p, hsa-miR-606, hsa-miR-150, hsa-miR-4776-5p, hsa-miR-3124-5p, hsa-miR-4741, hsa-miR-635, hsa-miR-4668-5p, hsa-miR-4763-3p, hsa-miR-4417, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

b) hsa-miR-223, hsa-miR-142-3p, hsa-miR-146b, hsa-miR-21

c) hsa-miR-3201, hsa-miR-4423-3p, hsa-miR-223, hsa-miR-3128, hsa-miR-155, hsa-miR-3163, hsa-miR-146b-5p, hsa-miR-606, hsa-miR-150, hsa-miR-4776-5p, hsa-miR-142-3p, hsa-miR-21, hsa-miR-3124-5p, hsa-miR-4741, hsa-miR-635, hsa-miR-4668-5p, hsa-miR-4763-3p, hsa-miR-4417, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

d) hsa-miR-3201, hsa-miR-4423-3p, hsa-miR-3128, hsa-miR-3163, hsa-miR-606, hsa-miR-4776-5p, hsa-miR-635, hsa-miR-3124-5p, hsa-miR-4741, hsa-miR-4668-5p, hsa-miR-4763-3p, hsa-miR-1911*, hsa-miR-4734, hsa-miR-4530, hsa-miR-4260, hsa-miR-3185, hsa-miR-4707-5p, hsa-miR-4745-5p, hsa-miR-4659a-3p, hsa-miR-1469, hsa-miR-4695-5p, hsa-miR-4674, hsa-miR-4687-3p, hsa-miR-4739, hsa-miR-1915, hsa-miR-216b, hsa-miR-548a-3p

e) hsa-miR-146b-5p.

6. The method of claim 1 comprising further determining in a sample from said subject the expression of one or more microRNAs selected from the group consisting of

1) hsa-miR-133a, hsa-miR-206, hsa-miR-133b, hsa-miR-1, hsa-miR-299-5p, hsa-miR-486-3p, hsa-miR-381, hsa-miR-4324, hsa-miR-154, hsa-miR-1247, hsa-miR-1287, hsa-miR-376c, hsa-miR-195*, hsa-miR-411, hsa-miR-4269, hsa-miR-328, hsa-miR-1296, hsa-miR-34c-3p, hsa-miR-204, hsa-miR-504, hsa-miR-30c-1*, hsa-miR-615-3p, hsa-miR-654-3p, hsa-miR-505, hsa-miR-508-5p, hsa-miR-409-5p, hsa-miR-338-5p, hsa-miR-433, hsa-miR-375, hsa-miR-935, hsa-let-7d*, hsa-miR-99a*, hsa-miR-128, hsa-miR-1290, hsa-miR-331-5p, hsa-miR-4730, hsa-miR-29c*, hsa-miR-455-5p, hsa-miR-377*, hsa-miR-378e, hsa-miR-584, hsa-miR-96, hsa-miR-30a*, hsa-miR-143*, hsa-miR-370, hsa-miR-148a*, hsa-miR-489, hsa-miR-506, hsa-let-7e*, hsa-miR-30e*, hsa-miR-4787-3p, hsa-let-7b*, hsa-miR-181c*, hsa-miR-513a-5p, hsa-miR-92a-1*, hsa-miR-337-5p, hsa-miR-1181, hsa-miR-1237, hsa-miR-3620, hsa-miR-485-5p, hsa-miR-23c, hsa-miR-18a*, hsa-miR-10a, hsa-miR-139-3p, hsa-miR-493, hsa-miR-148b, hsa-miR-550a, hsa-miR-3147, hsa-miR-378g, hsa-miR-675*, hsa-miR-1238, hsa-miR-542-5p, hsa-miR-30c-2*, hsa-miR-125a-3p, hsa-miR-181a-2*, hsa-miR-1292, hsa-miR-187, hsa-miR-378*, hsa-miR-493*, hsa-miR-495, hsa-miR-557, hsa-miR-3909, hsa-miR-378d, hsa-miR-491-5p, hsa-miR-181d, hsa-miR-671-3p, hsa-miR-513c, hsa-miR-487b, hsa-miR-378b, hsa-miR-885-5p, hsa-miR-98, hsa-miR-29b-2*, hsa-miR-4685-3p, hsa-miR-3605-3p, hsa-miR-24-1*, hsa-miR-4649-3p, hsa-miR-3180-5p, hsa-miR-149, hsa-miR-23b*

2) hsa-miR-206, hsa-miR-133b, hsa-miR-133a, hsa-miR-1, hsa-miR-486-3p, hsa-miR-299-5p, hsa-miR-1247, hsa-miR-381, hsa-miR-154, hsa-miR-433, hsa-miR-1244, hsa-miR-4485, hsa-miR-378e, hsa-miR-505, hsa-miR-376c, hsa-miR-1296, hsa-miR-29b-1*, hsa-miR-409-5p, hsa-miR-29c*, hsa-miR-34c-3p, hsa-miR-4646-5p, hsa-miR-99a*, hsa-miR-378g, hsa-miR-504, hsa-miR-584, hsa-miR-128, hsa-miR-30c-1*, hsa-miR-665, hsa-miR-4269, hsa-miR-493*, hsa-miR-328, hsa-miR-550a, hsa-miR-375, hsa-miR-493, hsa-miR-378d, hsa-miR-148a*, hsa-miR-615-3p, hsa-miR-431*, hsa-miR-378i, hsa-miR-422a, hsa-miR-331-5p, hsa-miR-378*, hsa-miR-148b, hsa-miR-601, hsa-miR-4288, hsa-miR-596, hsa-miR-10a, hsa-miR-200c*, hsa-miR-378b, hsa-miR-378f, hsa-miR-4257, hsa-miR-370, hsa-miR-1237, hsa-miR-3942-3p

3) hsa-miR-211, hsa-miR-29b, hsa-miR-4324, hsa-miR-143*, hsa-miR-1287, hsa-miR-4708-5p, hsa-miR-195*, hsa-miR-508-5p, hsa-miR-204, hsa-miR-96, hsa-let-7b*, hsa-miR-935, hsa-miR-675*, hsa-miR-149, hsa-miR-30a*, hsa-miR-1181, hsa-miR-506, hsa-miR-23c, hsa-miR-127-5p, hsa-miR-491-5p

4) hsa-miR-30b, hsa-miR-221, hsa-miR-141, hsa-miR-339-3p, hsa-miR-100, hsa-miR-331, hsa-miR-598, hsa-miR-24, hsa-miR-23a, hsa-miR-99a, hsa-miR-152, hsa-miR-423-5p, hsa-miR-34c, hsa-miR-27a, hsa-miR-30c, hsa-miR-199a, hsa-miR-125a-5p, mmu-miR-491, hsa-miR-489, hsa-miR-337-5p, hsa-miR-149, hsa-miR-127, hsa-miR-375, hsa-miR-101, hsa-miR-296, mmu-miR-379, hsa-miR-574-3p, hsa-miR-487b, mmu-miR-499, hsa-miR-95, hsa-miR-885-5p, hsa-miR-486-3p, hsa-miR-1, hsa-miR-133b, hsa-miR-133a.

a) hsa-miR-133a, hsa-miR-133b, hsa-miR-1, hsa-miR-299-5p, hsa-miR-486-3p, hsa-miR-381, hsa-miR-4324, hsa-miR-154, hsa-miR-1247, hsa-miR-1287, hsa-miR-376c, hsa-miR-195*, hsa-miR-4269, hsa-miR-1296, hsa-miR-34c-3p, hsa-miR-204, hsa-miR-504, hsa-miR-30c-1*, hsa-miR-615-3p, hsa-miR-505, hsa-miR-508-5p, hsa-miR-409-5p, hsa-miR-433, hsa-miR-375, hsa-miR-935, hsa-miR-128, hsa-miR-331-5p, hsa-miR-584, hsa-miR-30a*, hsa-miR-370, hsa-miR-148a*, hsa-miR-489, hsa-miR-30e*, hsa-miR-337-5p, hsa-miR-1181, hsa-miR-1237, hsa-miR-485-5p, hsa-miR-23c, hsa-miR-148b, hsa-miR-378g, hsa-miR-1238, hsa-miR-181a-2*, hsa-miR-187, hsa-miR-378d, hsa-miR-181 d, hsa-miR-98, hsa-miR-4685-3p, hsa-miR-23b*

b) hsa-miR-331, hsa-miR-598, hsa-miR-24, hsa-miR-23a, hsa-miR-99a, hsa-miR-152, hsa-miR-423-5p, hsa-miR-34c, hsa-miR-27a, hsa-miR-30c, hsa-miR-125a-5p, mmu-miR-491, hsa-miR-127, hsa-miR-375, hsa-miR-101, hsa-miR-296, mmu-miR-379, hsa-miR-574-3p, hsa-miR-487b, mmu-miR-499, hsa-miR-95, hsa-miR-885-5p, hsa-miR-486-3p, hsa-miR-1, hsa-miR-133b, hsa-miR-133a

c) hsa-miR-133a, hsa-miR-133b, hsa-miR-1, hsa-miR-299-5p, hsa-miR-486-3p, hsa-miR-381, hsa-miR-4324, hsa-miR-154, hsa-miR-1247, hsa-miR-885-5p, hsa-miR-1287, hsa-miR-95, hsa-miR-376c, hsa-miR-195*, hsa-miR-4269, mmu-miR-499, hsa-miR-1296, hsa-miR-34c-3p, hsa-miR-204, hsa-miR-504, hsa-miR-30c-1*, hsa-miR-487b, hsa-miR-615-3p, hsa-miR-505, hsa-miR-508-5p, hsa-miR-409-5p, hsa-miR-433, hsa-miR-375, hsa-miR-935, hsa-miR-128, hsa-miR-331-5p, hsa-miR-584, hsa-miR-30a*, hsa-miR-370, hsa-miR-148a*, hsa-miR-489, hsa-miR-30e*, hsa-miR-337-5p, hsa-miR-1181, hsa-miR-1237, hsa-miR-485-5p, hsa-miR-23c, hsa-miR-491-5p, hsa-miR-148b, hsa-miR-378g, hsa-miR-125a-5p, hsa-miR-1238, hsa-miR-181a-2*, hsa-miR-187, hsa-miR-30c, hsa-miR-27a, hsa-miR-99a, hsa-miR-378d, hsa-miR-181d, hsa-miR-98, hsa-miR-4685-3p, hsa-miR-598, hsa-miR-23b*

d) hsa-miR-133a, hsa-miR-133b, hsa-miR-1, hsa-miR-486-3p, hsa-miR-34c-3p, hsa-miR-375.

7. Method according to one of claims 1 to 6, wherein the sample is a skin biopsy taken from the affected skin areas.

8. A method for monitoring or determining the efficacy of a treatment of rosacea type II in a subject comprising determining in a dermal and/or epidermal sample from said subject the expression of at least one combination of the seven following microRNAs: hsa-miR-3201, hsa-miR-4423-3p, hsa-miR-3128, hsa-miR-3163, hsa-miR-606, hsa-miR-4776-5p, and hsa-miR-635, and the decreased expression or activity of microRNAs at the end of or during treatment indicating the efficacy of the treatment.

9. The method of claim 8, wherein the sample is a skin biopsy taken from the affected skin areas.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **ZIBERT et al.** *J Dermatol Sci.,* 2010, vol. 58 (3), 177-85

- **BENJAMINI ; HOCHBERG.** Methodological. *Journal of the Royal Statistical Society,* 1995, vol. 57 (1), 289-300